# EUROPEAN PATENT SPECIFICATION

(11) **EP 1 636 175 B1**
(45) Date of publication and mention of the grant of the patent: **15.10.2008**
(21) Application number: 04726045.0
(22) Date of filing: 06.04.2004
(51) Int. Cl.: C07C 317/22, C07C 59/48, A61K 31/10, A61K 31/192

(54) **PHENOXYACETIC ACID DERIVATIVES FOR TREATING RESPIRATORY DISORDERS**
PHENOXYESSIGSÄURE-DERIVATE ZUR BEHANDLUNG VON STÖRUNGEN DER ATEMWEGE
DERIVES D'ACIDE PHENOXYACETIQUE POUR TRAITER LES TROUBLES RESPIRATOIRES

(30) Priority: 07.04.2003 SE 0301009
(43) Date of publication of application: 22.03.2006
(73) Proprietor: AstraZeneca AB, 151 85 Södertälje (SE)
(72) Inventor: PAIRAUDEAU, Garry, Loughborough, Leicestershire LE11 5RH (GB); MOHAMMED, Rukhsana, Loughborough, Leicestershire LE11 5RH (GB); THOM, Stephen, Loughborough, Leicestershire LE11 5RH (GB)
(86) International application number: PCT/SE2004/000534
(87) International publication number: WO 2004/089884

(56) References cited:
- EP-A2- 1 170 594
- WO-A1-01/60807
- GB-A- 1 356 834

## Description

The present invention relates to substituted phenoxyacetic acids as useful pharmaceutical compounds for treating respiratory disorders, pharmaceutical compositions containing them, and processes for their preparation.

EPA 1 170 594 discloses methods for the identification of compounds useful for the treatment of disease states mediated by prostaglandin D2, a ligand for orphan receptor CRTH2. GB 1356834 discloses a series of compounds said to possess anti-inflammatory, analgesic and antipyretic activity. It has been found that certain phenoxyacetic acids are active at the CRTH2 receptor, and as a consequence are expected to be potentially useful for the treatment of various respiratory diseases, including asthma and COPD.

In a first aspect the invention therefore provides a compound of formula (I) or a pharmaceutically acceptable salt or solvate thereof: 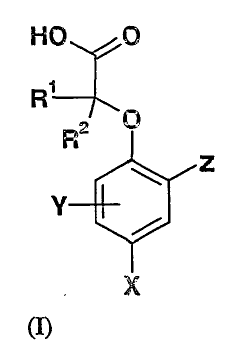 in which
X is C₁₋₆alkyl, OR⁶ or OR¹⁷
Y is selected from hydrogen, halogen, CN, nitro, SO₂R³, OR⁴, SR⁴, SOR³, SO₂NR⁴R⁵, CONR⁴R⁵, NR⁴R⁵, NR⁶SO₂R¹, NR⁶CO₂R⁶, NR⁶COR³, C₂-C₆ alkenyl, C₂-C₆ alkynyl, C₃-C₇ cycloalkyl or C₁₋₆alkyl, the latter four groups being optionally substituted by one or more substituents independently selected from halogen, OR⁶ and NR⁶R⁷, S(O)ₙR⁶ where n is 0, 1 or 2;
Z is aryl or a ring A, where A is a six membered heterocyclic aromatic ring containing one or more nitrogen atoms or may be a 6,6 or 6,5 fused bicycle containing one or more O, N, S atoms, the aryl or A rings all being optionally substituted by one or more substituents independently selected from from hydrogen, halogen, CN, OH, SH, nitro, COR⁹, CO₂R⁶, SO₂R⁹, OR⁹, SR⁹, SOR⁹, SO₂NR¹⁰R¹¹, CONR¹⁰R¹¹, NR¹⁰R¹¹, NHSO₂R⁹, NR⁹SO₂R⁹, NR⁶CO₂R⁶, NHCOR⁹, NR⁹COR⁹, NR⁶CONR⁴R⁵, NR⁶SO₂NR⁴R⁵, aryl, heteroaryl, C₂-C₆ alkenyl, C₂-C₆ alkynyl, C₃-C₇ cycloalkyl or C₁₋₆alkyl, the latter four groups being optionally substituted by one or more substituents independently selected from halogen, C₃-C₇ cycloalkyl, OR⁶, NR⁶R⁷, S(O)ₙR⁶ (where n is 0, 1 or 2), CONR⁶R⁷, NR⁶COR⁷, SO₂NR⁶R⁷ and NR⁶SO₂R⁷;
R¹ and R² independently represent a hydrogen atom, halogen, C₂-C₆ alkenyl, C₂-C₆ alkynyl, C₃-C₇ cycloalkyl or a C₁₋₆alkyl group, the latter four groups being optionally substituted by one or more substituents independently selected from halogen, C₃-C₇ cycloalkyl, NR⁶R⁷, OR⁶, S(O)ₙR⁶ (where n is 0, 1 or 2);
or
R¹ and R² together can form a 3-8 membered ring optionally containing one or more atoms selected from O, S, NR⁶ and itself optionally substituted by one or more C₁-C₃ alkyl or halogen;
R³ represents C₃-C₇ cycloalkyl or C₁₋₆alkyl which may be optionally substituted by one or more substituents independently selected from halogen, C₃-C₇ cycloalkyl, OR⁶ and NR⁶R⁷, S(O)ₙR⁶ (where n = 0,1 or 2), CONR⁶R⁷, NR⁶COR⁷,SO₂NR⁶R⁷ and NR⁶SO₂R⁷;
R⁴ and R⁵ independently represent hydrogen, C₃-C₇ cycloalkyl or C₁₋₆alkyl, the latter two groups being optionally substituted by one or more substituents independently selected from halogen, C₃-C₇ cycloalkyl, OR⁶ and NR⁶R⁷, S(O)ₙR⁶ (where n = 0, 1 or 2), CONR⁶R⁷, NR⁶COR⁷,SO₂NR⁶R⁷ and NR⁶SO₂R⁷;
or
R⁴ and R⁵ together with the nitrogen atom to which they are attached can form a 3-8 membered saturated heterocylic ring optionally containing one or more atoms selected from O, S(O)ₙ (where n = 0,1 or 2), NR⁸, and itself optionally substituted by halogen or C₁₋₃ alkyl;
R⁶ and R⁷ independently represents a hydrogen atom or C₁-C₆ alkyl;
R⁸ is hydrogen, C₁₋₄ alkyl, -COC₁-C₄ alkyl, CO₂C₁-C₄alkyl or CONR⁶C₁-C₄alkyl;
R⁹ represents aryl, heteroaryl, C₃-C₇ cycloalkyl or C₁₋₆alkyl, the latter two groups may be optionally substituted by one or more substituents independently selected from halogen, C₃-C₇ cycloalkyl, aryl, heteroaryl OR⁶ and NR⁶R⁷, S(O)ₙR⁶ (where n = 0, 1 or 2), CONR⁶R⁷, NR⁶COR⁷, SO₂NR⁶R⁷ and NR⁶SO₂R⁷;
R¹⁰ and R¹¹ independently represent aryl or heteroaryl, hydrogen, C₃-C₇ cycloalkyl or C₁₋₆alkyl, the latter two groups being optionally substituted by one or more substituents independently selected from halogen, C₃-C₇ cycloalkyl, aryl, heteroaryl, OR⁶ and NR⁶R⁷,
S(O)ₙR⁶ (where n = 0, 1 or 2), CONR⁶R⁷, NR⁶COR⁷, SO₂NR⁶R⁷ and NR⁶SO₂R⁷;
or
R¹⁰ and R¹¹ together with the nitrogen atom to which they are attached can form a 3-8 membered saturated heterocylic ring optionally containing one or more atoms selected from O, S(O)ₙ (where n = 0, 1 or 2), NR⁸, and itself optionally substituted by halogen or C₁-C₃ alkyl.
R¹⁷ is C₁₋₆ alkyl which is substituted by one or more halogen atoms;
Examples of aryl include phenyl and naphthyl.

Heteroaryl is defined as a 5-7 member aromatic ring or can be 6,6- or 6,5-fused bicyclic ring optionally containing one or more heteroatoms selected from N, S or O. The bicyclic ring may be linked through carbon or nitrogen and may be attached through the 5 or 6 membered ring and can be fully or partially saturated. Examples include pyridine, pyrimidine, thiazole, oxazole, pyrazole, imidazole, furan, isoxazole, pyrrole, isothiazole and azulene, naphthyl, indene, quinoline, isoquinoline, indole, indolizine, benzo[b]furan, benzo[b]thiophene, 1H-indazole, benzimidazole, benzthiazole, benzoxazole, purine, 4H-quinolizine, cinnoline, phthalazine, quinazoline, quinoxaline, 1,8-naphthyridine, pteridine, quinolone and 1,2-methylenedioxy benzene.

Aryl or heteroaryl groups can be optionally substituted by one or more substituents independently selected from from hydrogen, halogen, CN, OH, SH, nitro, CO₂R⁶, SO₂R⁹, OR⁹, SR⁹, SOR⁹, SO₂NR¹⁰R¹¹, CONR¹⁰R¹¹, NR¹⁰R¹¹, NHSO₂R⁹, NR⁹SO₂R⁹, NR⁶CO₂R⁶, NHCOR⁹, NR⁹COR⁹, aryl, heteroaryl, C₂-C₆ alkenyl, C₂-C₆ alkynyl, C₃-C₇ cycloalkyl or C₁₋₆alkyl, the latter four groups being optionally substituted by one or more substituents independently selected from halogen, C₃-C₇ cycloalkyl , OR⁶, NR⁶R⁷ S(O)ₙR⁶ (where n is 0, 1 or 2), CONR⁶R⁷, NR⁶COR⁷, SO₂NR⁶R⁷ and NR⁶SO₂R⁷.

The group A is a six membered heterocyclic ring containing one or more nitrogen atoms or may be a 6,6 or 6,5 fused bicycle containing one or more O, N, S atoms. Examples of suitable rings include pyridine, pyrimidine, pyrazine, pyridazine, indole, quinoline, isoquinoline, benzimidazole, benzthiazole, benzofuran, benzoxazole, benzthiophene, phthalazine and quinazoline.

In the context of the present specification, unless otherwise indicated, an alkyl or alkenyl group or an alkyl or alkenyl moiety in a substituent group may be linear or branched.

Heterocyclic rings as defined for R⁴, R⁵ and R¹⁰, R¹¹ means saturated heterocycles, examples include morpholine, azetidine, pyrrolidine, piperidine and piperazine. Substuitents can be present on carbon or appropriate nitrogen atoms of said rings.

Preferably X is C₁₋₄alkyl or C₁₋₄alkoxy, more preferably methyl, ethyl or methoxy.

Preferably Y is hydrogen.

Preferably Z is phenyl optionally substituted as defined above. One or more substitents can be present. Preferred substituents for all Z groups include those substituents exemplified herein, in particular halogen,C₁₋₃alkyl, cyano and SO₂R⁹. Most preferably Z is phenyl or phenyl substituted by a single substituent selected from SO₂Me, SO₂Et or CN or di-substituted by SO₂Et and methyl.

Preferably R¹ and R² are independently hydrogen or C₁₋₃ alkyl. More preferably R¹ and R² are both hydrogen or one is hydrogen and the other is methyl.

Preferred compounds of the invention include:
[(5-Methylbiphenyl-2-yl)oxy]acetic acid,
{[5-Ethyl-4'-(methylsulfonyl)biphenyl-2-yl]oxy} acetic acid
{[4'-(Ethylsulfonyl)-5-methoxybiphenyl-2-yl]oxy} acetic acid
[[4-Chloro-4'-(ethylsulfonyl)-2',5-dimethyl[1,1'-biphenyl]-2-yl]oxy]-acetic acid
[[4'-(Ethylsulfonyl)-2',5-dimethyl[1,1'-biphenyl]-2-yl]oxy]-acetic acid
2-[[3'-Cyano-5-methyl[1,1'-biphenyl]-2-yl]oxy]-(2S)-propanoic acid
2-[[2'-Fluoro-5'-cyano-5-methyl[1,1'-biphenyl]-2-yl]oxy]-(2S)-propanoic acid
and pharmaceutically acceptable salts and solvates thereof.

Certain compounds of formula (I) are capable of existing in stereoisomeric forms. It will be understood that the invention encompasses all geometric and optical isomers of the compounds of formula (I) and mixtures thereof including racemates. Tautomers and mixtures thereof also form an aspect of the present invention.

The compound of formula (I) above may be converted to a pharmaceutically acceptable salt or solvate thereof, preferably a basic addition salt such as sodium, potassium, calcium, aluminium, lithium, magnesium, zinc, benzathine, chloroprocaine, choline, diethanolamine, ethanolamine, ethyldiamine, meglumine, tromethamine or procaine, or an acid addition salt such as a hydrochloride, hydrobromide, phosphate, acetate, fumarate, maleate, tartrate, citrate, oxalate, methanesulphonate or p-toluenesulphonate.

It will be appreciated by those skilled in the art that in the processes of the present invention certain functional groups in the starting reagents or intermediate compound may need to be protected by protecting groups. Thus, the preparation of the compound of formula (I) may involve, at an appropriate stage, the removal of one or more protecting groups. The protection and deprotection of functional groups is fully described in 'Protective Groups in Organic Chemistry', edited by J. W. F. McOmie, Plenum Press (1973), and 'Protective Groups in Organic Synthesis', 3rd edition, T. W. Greene & P. G. M. Wuts, Wiley-Interscience (1999).

Compounds of formula (I) can be prepared by reaction of a compound of formula (II): 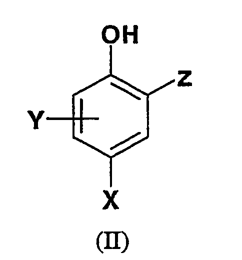 in which X, Y and Z are as defined in formula (I) or are protected derivatives thereof, with a compound of formula (III):

L-CR¹R²CO₂R¹² (III)

Where R¹ and R² are as defined in formula (I) or are protected derivatives thereof, R¹² is H or C₁-C₁₀ alkyl group and L is a leaving group, and optionally thereafter in any order:
- removing any protecting group
- hydrolysing the ester group R¹² to the corresponding acid
- oxidation of sulphides to sulphoxides or sulphones
- forming a pharmaceutically acceptable salt or solvate.

The reaction can be carried out in a suitable solvent such as DMF using a base such as potassium carbonate or the like. Suitable groups R¹² include C₁₋₆ alkyl groups such as methyl, ethyl or tert-butyl. Suitable L is a leaving group such as halo, in particular chlorine or bromine. L may also be hydroxy so that a Mitsunobu reaction may be performed with compound (II) using for example triphenylphosphine and diethyl azodicarboxylate.

Hydrolysis of the ester group R¹² can be carried out using routine procedures, for example treatment of methyl and ethyl esters with aqueous sodium hydroxide, and treatment of tert-butyl esters with acids such as trifluoroacetic acid.

Compounds of formula (II) can be prepared by reaction of a compound of formula (IV) with a compound of formula (V) *via* a Suzuki coupling reaction followed by deprotection of R¹³ when R¹³ is not equal to H : 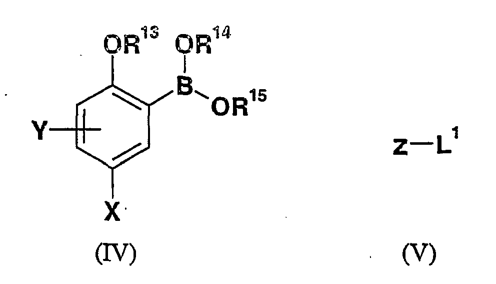 in which X, Y and Z are as defined in formula (I) or are protected derivatives thereof, R¹³ is H or a suitable protecting group, for example benzyl, L¹ is iodide, bromide, chloride or triflate and R¹⁴ and R¹⁵ are H or C₁-C₆ alkyl groups or R¹⁴ and R¹⁵ together can form a 5 or 6 membered ring optionally substituted by one or more C₁-C₃ alkyl.

The reaction can be carried out in a suitable solvent such as dioxane using a palladium catalyst such as [1,1-bis(diphenylphosphino)ferrocene]dichloropalladium and a base such as cesium fluoride, preferably at elevated temperatures.

Compounds of formula (IV) can be prepared from a compound of formula (VI) by formation of an organometallic (VII) followed by reaction with a borate ester, as outlined in Scheme I. 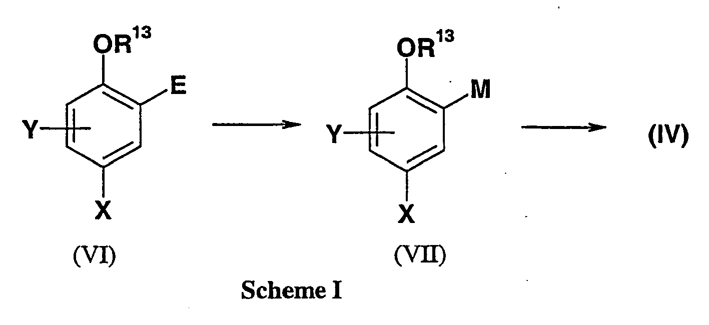 in which X, Y are as defined in formula (I) or are protected derivatives thereof, R¹³ is as defined in formula (IV), E is hydrogen or halogen and M is a metal such as Na or Li. For example when R¹³ is benzyl and E is bromine, butyl lithium can be used to form the intermediate (VII) where M = Li. The reaction is performed at -78°C in diethylether, then quenched with a borate ester such as trimethylborate.

Compounds of formula (IV) may also be prepared by a palladium catalysed coupling of compounds of formula (VIII) with a suitable boronic ester, for example (IX) or (X). 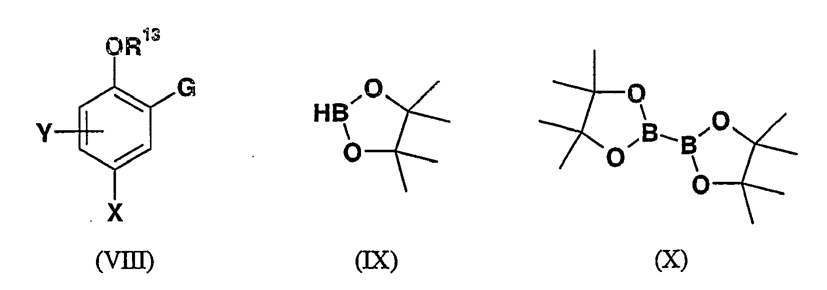 in which X, Y and R¹³ are as defined above and G is halogen or triflate

Compounds of formula (II) may also be prepared by reaction of a compound of formula (XI) with a compound of formula (XII) using Suzuki coupling methodology. 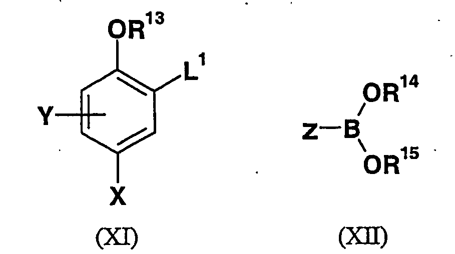 in which X, Y, Z, R¹³, L¹, R¹⁴ and R¹⁵ are as defined above and compounds of formula (XI) and (XII) can be made using the same methodology as above.

Compounds of formula (II), where Z=heteroaryl may also be prepared by ring synthesis, for example a compound of formula (XIII) may be formed by reaction of a compound of formula (XIV) with a compound of formula (XV).
X, Y and R¹³ are as defined above and R¹⁶ is as defined as a substituent on Z as defined in formula (I) or are protected derivatives thereof. The reaction can be carried out in a solvent such as ethanol under reflux, and a base such as sodium ethoxide can be used if compound of formula (XV) is a salt

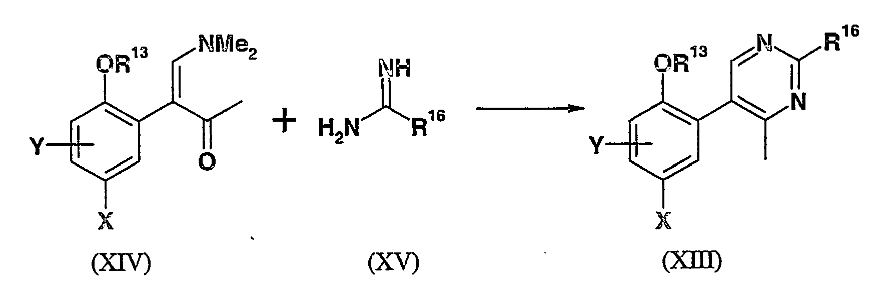

When R¹⁶ is a group S-alkyl, this may be further elaborated by oxidation to the sulfoxide or sulphone using an oxidizing agent such as mcpba in DCM at RT. This may then be displaced with an appropriate nucleophile as defined for Z in formula 1. Scheme 2;

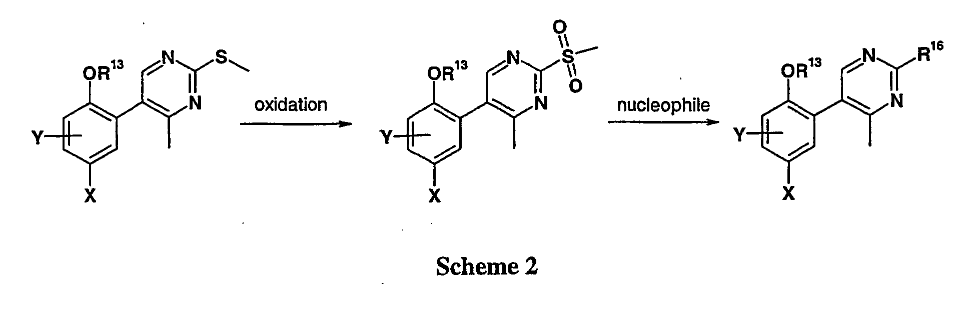

The sequence of the steps above may be changed, for example a compound of formula (XVI) may be formed by the reaction of a compound of formula (XVII) with a compound of formula (XII) using a Suzuki coupling.

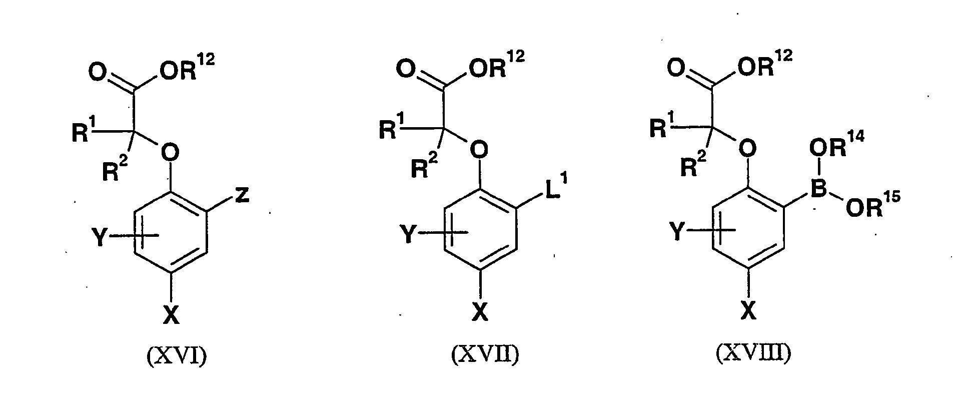

Compounds of formula (I) may also be prepared by reaction of a compound of formula (XVIII) in which in which X, Y, R¹, R², R¹²,R¹⁴ and R¹⁵ are as defined above with a compound of formula (V) using Suzuki coupling method as defined above.

A compound of formula (XVIII) may be prepared by method A or B

### Method A

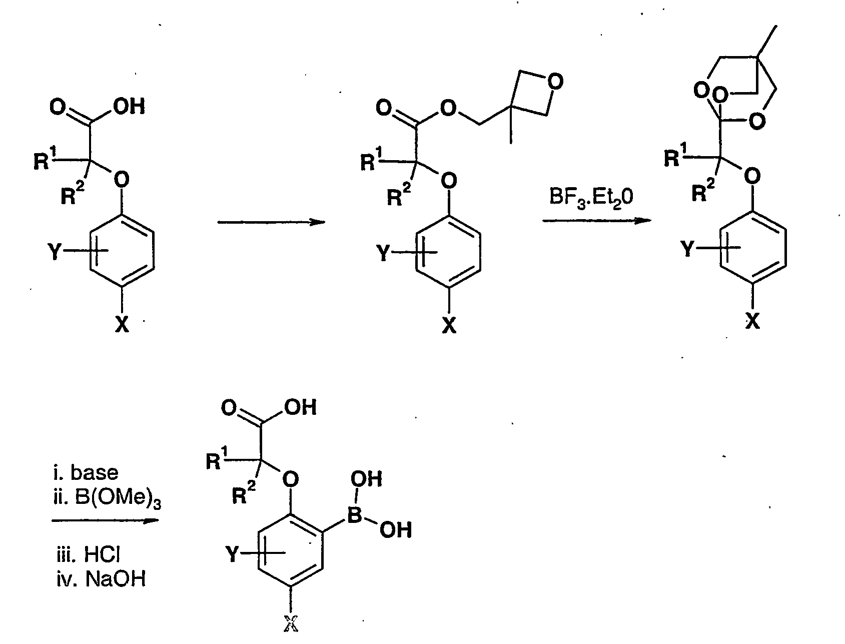

The acid was first converted to the acid chloride, using for example oxalylchloride in DCM at RT, then reacted with 3-methyl-3-oxetanemethanol in the presence of a base such as triethylamine to give the ester. The oxetane ester is the rearranged to the OBO ester using boron trifluoride diethyletherate in DCM at -78°C to RT. Deprotonation with a base such as sec -butyl lithium at low temperature followed by quenching with trimethylborate gave the protected diacid which was then deprotected using HCl then sodium hydroxide

### Method B

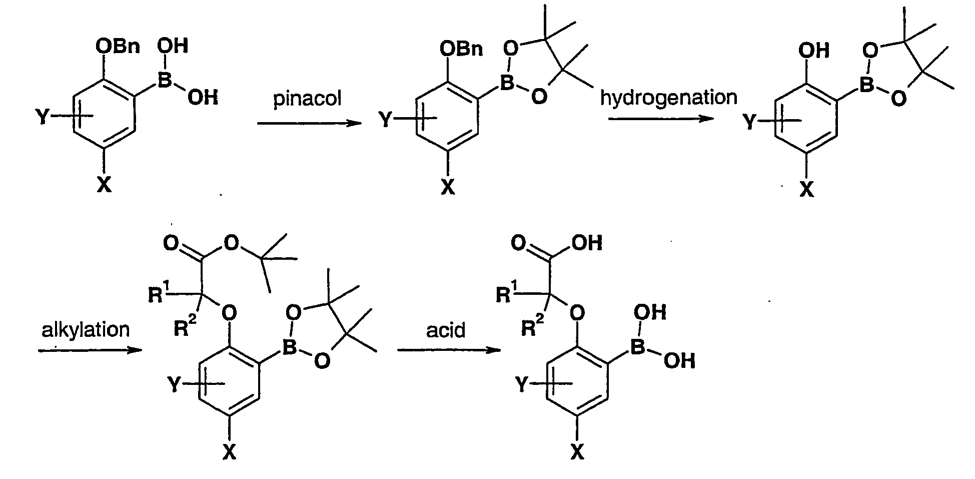

A compound of formula (IV) where R¹³ = Bn and R¹⁴ and R¹⁵= H and pinacol can bestirred at RT in a suitable solvent such as diethylether to give the boronate ester. The benzyl group may be removed by hydrogenation at RT using palladium on carbon as catalyst then alkylated with a compound of formula (III) using a base or mitsunobu conditions. Treatment with acid such as HCl or trifluoroacetic acid then removes the protecting groups.

In a further aspect, the present invention provides the use of a compound of formula (I), a pharmaceutically acceptable salt or solvate thereof for use in therapy.

The compounds of formula (I) have activity as pharmaceuticals, in particular as modulators of CRTh2 receptor activity, and may be used in the treatment (therapeutic or prophylactic) of conditions/diseases in human and non-human animals which are exacerbated or caused by excessive or unregulated production of PGD₂ and its metabolites. Examples of such conditions/diseases include:
(1) **(the respiratory tract)** obstructive airways diseases including: asthma (such as bronchial, allergic, intrinsic, extrinsic and dust asthma particularly chronic or inveterate asthma (e.g. late asthma and airways hyper-responsiveness)), chronic obstructive pulmonary disease (COPD)(such as irreversible COPD) ; bronchitis (including eosinophilic bronchitis); acute, allergic, atrophic rhinitis or chronic rhinitis (such as rhinitis caseosa, hypertrophic rhinitis, rhinitis purulenta, rhinitis sicca), rhinitis medicamentosa, membranous rhinitis (including croupous, fibrinous and pseudomembranous rhinitis), scrofoulous rhinitis, perennial allergic rhinitis, easonal rhinitis (including rhinitis nervosa (hay fever) and vasomotor rhinitis); nasal polyposis; sarcoidosis; farmer's lung and related diseases; fibroid lung; idiopathic interstitial pneumonia; cystic fibrosis; antitussive activity; treatment of chronic cough associated with inflammation or iatrogenic induced ;
(2) **(bone and joints)** arthrides including rheumatic, infectious, autoimmune, seronegative, spondyloarthropathies (such as ankylosing spondylitis, psoriatic arthritis and Reiter's disease), Behcet's disease, Sjogren's syndrome and systemic sclerosis;
(3) **(skin and eyes)** psoriasis, atopical dermatitis, contact dermatitis, other eczmatous dermitides, seborrhoetic dermatitis, Lichen planus, Pemphigus, bullous Pemphigus, Epidermolysis bullosa, urticaria, angiodermas, vasculitides, erythemas, cutaneous eosinophilias, chronic skin ulcers, uveitis, Alopecia areatacorneal ulcer and vernal conjunctivitis;
(4) **(gastrointestinal tract)** Coeliac disease, proctitis, eosinopilic gastro-enteritis, mastocytosis, Crohn's disease, ulcerative colitis, irritable bowel disease; food-related allergies which have effects remote from the gut, (such as migraine, rhinitis and eczema);
(5) **(central and peripheral nervous system)** Neurodegenerative diseases and dementia disorders (such as Alzheimer's disease, amyotrophic lateral sclerosis and other motor neuron diseases, Creutzfeldt-Jacob's disease and other prion diseases, HIV encephalopathy (AIDS dementia complex), Huntington's disease, frontotemporal dementia, Lewy body dementia and vascular dementia), polyneuropathies (such as Guillain-Barré syndrome, chronic inflammatory demyelinating polyradiculoneuropathy, multifocal motor neuropathy), plexopathies, CNS demyelination (such as multiple sclerosis, acute disseminated/haemorrhagic encephalomyelitis, and subacute sclerosing panencephalitis), neuromuscular disorders (such as myasthenia gravis and Lambert-Eaton syndrome), spinal diorders (such as tropical spastic paraparesis, and stiff-man syndrome), paraneoplastic syndromes (such as cerebellar degeneration and encephalomyelitis), CNS trauma, migraine and stroke.
(6) **(other tissues and systemic disease)** atherosclerosis, acquired Immunodeficiency Syndrome (AIDS), lupus erythematosus; systemic lupus, erythematosus; Hashimoto's thyroiditis, type I diabetes, nephrotic syndrome, eosinophilia fascitis, hyper IgE syndrome, lepromatous leprosy, idiopathic thrombocytopenia pupura; post-operative adhesions, sepsis and ischemic/reperfusion injury in the heart, brain, peripheral limbs hepatitis (alcoholic, steatohepatitis and chronic viral) , glomerulonephritis, renal impairment, chronic renal failure and other organs
(7) **(allograft rejection)** acute and chronic following, for example, transplantation of kidney, heart, liver, lung, bone marrow, skin and cornea; and chronic graft versus host disease;
(8) Diseases associated with raised levels of PGD₂ or its metabolites.

(1) (respiratory tract) - obstructive diseases of the airways including: asthma, including bronchial, allergic, intrinsic, extrinsic, exercise-induced, drug-induced (including aspirin and NSAID-induced) and dust-induced asthma, both intermittent and persistent and of all severities, and other causes of airway hyper-responsiveness ; chronic obstructive pulmonary disease (COPD) ; bronchitis , including infectious and eosinophilic bronchitis; emphysema; bronchiectasis; cystic fibrosis; sarcoidosis; farmer's lung and related diseases; hypersensitivity pneumonitis; lung fibrosis, including cryptogenic fibrosing alveolitis, idiopathic interstitial pneumonias, fibrosis complicating anti-neoplastic therapy and chronic infection, including tuberculosis and aspergillosis and other fungal infections; complications of lung transplantation; vasculitic and thrombotic disorders of the lung vasculature, and pulmonary hypertension; antitussive activity including treatment of chronic cough associated with inflammatory and secretory conditions of the airways, and iatrogenic cough; acute and chronic rhinitis including rhinitis medicamentosa, and vasomotor rhinitis; perennial and seasonal allergic rhinitis including rhinitis nervosa (hay fever); nasal polyposis; acute viral infection including the common cold, and infection due to respiratory syncytial virus, influenza, coronavirus (including SARS) and adenovirus.
(2) (bone and joints) arthritides associated with or including osteoarthritis/osteoarthrosis, both primary and secondary to e.g. congenital hip dysplasia; cervical and lumbar spondylitis, and low back and neck pain; rheumatoid arthritis and Still's disease; seronegative spondyloarthropathies including ankylosing spondylitis, psoriatic arthritis, reactive arthritis and undifferentiated spondarthropathy; septic arthritis and other infection-related arthopathies and bone disorders such as tuberculosis, including Potts' disease and Poncet's syndrome; acute and chronic crystal-induced synovitis including urate gout, calcium pyrophosphate deposition disease, and calcium apatite related tendon, bursal and synovial inflammation; Behcet's disease; primary and secondary Sjogren's syndrome; systemic sclerosis and limited scleroderma; systemic lupus erythematosus, mixed connective tissue disease, and undifferentiated connective tissue disease; inflammatory myopathies including dermatomyositits and polymyositis; polymalgia rheumatica; juvenile arthritis including idiopathic inflammatory arthritides of whatever joint distribution and associated syndromes, and rheumatic fever and its systemic complications; vasculitides including giant cell arteritis, Takayasu's arteritis, Churg-Strauss syndrome, polyarteritis nodosa, microscopic polyarteritis, and vasculitides associated with viral infection, hypersensitivity reactions, cryoglobulins, and paraproteins; low back pain; Familial Mediterranean fever, Muckle-Wells syndrome, and Familial Hibernian Fever, Kikuchi disease; drug-induced arthalgias, tendonititides, and myopathies.
(3) (skin) psoriasis, atopic dermatitis, contact dermatitis or other eczematous dermatoses, and delayed-type hypersensitivity reactions; phyto- and photodermatitis; seborrhoeic dermatitis, dermatitis herpetiformis, lichen planus, lichen sclerosus et atrophica, pyoderma gangrenosum, skin sarcoid, discoid lupus erythematosus, pemphigus, pemphigoid, epidermolysis bullosa, urticaria, angioedema, vasculitides, toxic erythemas, cutaneous eosinophilias, alopecia areata, male-pattern baldness, Sweet's syndrome, Weber-Christian syndrome, erythema multiforme; cellulitis, both infective and non-infective; panniculitis;cutaneous lymphomas, non-melanoma skin cancer and other dysplastic lesions; drug-induced disorders including fixed drug eruptions.
(4) (eyes) blepharitis; conjunctivitis, including perennial and vernal allergic conjunctivitis; iritis; anterior and posterior uveitis; choroiditis; autoimmune; degenerative or inflammatory disorders affecting the retina; ophthalmitis including sympathetic ophthalmitis; sarcoidosis; infections including viral , fungal, and bacterial.
(5) (gastrointestinal tract) glossitis, gingivitis, periodontitis; oesophagitis, including reflux; eosinophilic gastro-enteritis, mastocytosis, Crohn's disease, colitis including ulcerative colitis, proctitis, pruritis ani; coeliac disease, irritable bowel syndrome, and food-related allergies which may have effects remote from the gut (for example migraine, rhinitis or eczema).
(6) (abdominal) hepatitis, including autoimmune, alcoholic and viral; fibrosis and cirrhosis of the liver; cholecystitis; pancreatitis, both acute and chronic.
(7) (genitourinary) nephritis including interstitial and glomerulonephritis; nephrotic syndrome; cystitis including acute and chronic (interstitial) cystitis and Hunner's ulcer; acute and chronic urethritis, prostatitis, epididymitis, oophoritis and salpingitis; vulvo-vaginitis; Peyronie's disease; erectile dysfunction (both male and female).
(8) (Allograft rejection) acute and chronic following, for example, transplantation of kidney, heart, liver, lung, bone marrow, skin or cornea or following blood transfusion; or chronic graft versus host disease;
(9) (CNS) Alzheimer's disease and other dementing disorders including CJD and nvCJD; amyloidosis; multiple sclerosis and other demyelinating syndromes; cerebral atherosclerosis and vasculitis; temporal arteritis; myasthenia gravis; acute and chronic pain (acute, intermittent or persistent, whether of central or peripheral origin) including visceral pain, headache, migraine, trigeminal neuralgia, atypical facial pain, joint and bone pain, pain arising from cancer and tumor invasion, neuropathic pain syndromes including diabetic, post-herpetic, and HIV-associated neuropathies; neurosarcoidosis; central and peripheral nervous system complications of malignant, infectious or autoimmune processes.
(10) Other auto-immune and allergic disorders including Hashimoto's thyroiditis, Graves' disease, Addison's disease, diabetes mellitus, idiopathic thrombocytopaenic purpura, eosinophilic fasciitis, hyper-IgE syndrome, antiphospholipid syndrome.
(11) Other disorders with an inflammatory or immunological component; including acquired immune deficiency syndrome (AIDS), leprosy, Sezary syndrome, and paraneoplastic syndromes.
(12) (Cardiovascular); atherosclerosis, affecting the coronary and peripheral circulation; pericarditis; myocarditis , inflammatory and auto-immune cardiomyopathies including myocardial sarcoid; ischaemic reperfusion injuries; endocarditis, valvulitis, and aortitis including infective (e.g. syphilitic); vasculitides; disorders of the proximal and peripheral veins including phlebitis and thrombosis, including deep vein thrombosis and complications of varicose veins.
(13) (Oncology) treatment of common cancers including prostate, breast, lung, ovarian, pancreatic, bowel and colon, stomach, skin and brain tumors and malignancies affecting the bone marrow (including the leukaemias) and lymphoproliferative systems, such as Hodgkin's and non-Hodgkin's lymphoma ; including the prevention and treatment of metastatic disease and tumour recurrences, and paraneoplastic syndromes.
(14) Diseases associated with raised levels of PGD₂ or its metabolites.

Thus, the present invention provides a compound of formula (I), or a pharmaceutically-acceptable salt or solvate thereof, as hereinbefore defined for use in therapy.

Preferably the compounds of the invention are used to treat diseases in which the chemokine receptor belongs to the CRTh2 receptor subfamily.

Particular conditions which can be treated with the compounds of the invention are asthma, rhinitis and other diseases in which raised levels of PGD₂ or its metabolites. It is preferred that the compounds of the invention are used to treat asthma.

In a further aspect, the present invention provides the use of a compound of formula (I), oar a pharmaceutically acceptable salt or solvate thereof, as hereinbefore defined in the manufacture of a medicament for use in therapy.

In a further aspect, the present invention provides the use of a compound or formula (I), or a pharmaceutically acceptable salt or solvate thereof, as hereinbefore defined in the manufacture of a medicament for use in therapy in combination with drugs used to treat asthma and rhinitis (such as inhaled and oral steroids, inhaled β2-receptor agonists and oral leukotriene receptor antagonists).

The invention further relates to combination therapies wherein a compound of formula (1) or a pharmaceutically acceptable salt, solvate or *in vivo* hydrolysable ester thereof, or a pharmaceutical composition or formulation comprising a compound of formula (1) is administered concurrently or sequentially or as a combined preparation with another therapeutic agent of agents, for the treatment of one or more of the conditions listed.

In particular, for the treatment, of the inflammatory diseases rheumatoid arthritis, psoriasis, inflammatory bowel disease, COPD, asthma and allergic rhinitis the compounds of the invention may be combined with agents such as tumour necrosis factor alpha (TNF-α) inhibitors such as anti-TNF monoclonal antibodies (for example Remicade, CDP-870 and adalimumab) and TNF receptor immunoglobulin molecules (such as Enbrel); non-selective cyclo-oygenase (COX)-1 / COX-2 inhibitors whether applied topically or systemically (such as piroxicam, diclofenac, propionic acids such as naproxen, flubiprofen, fenoprofen, ketoprofen and ibuprofen fenamates such as mefenamic acid, indomethacin, sulindac, azapropazone, pyrazolones such as phenylbutazone, salicylates such as aspirin), COX-2 inhibitors (such as meloxicam, celecoxib, rofecoxib, valdecoxib, lumarocoxib, parecoxib and etoricoxib); glucocorticosteroids (whether administered by topical,oral, intramuscular, intravenous, or intra-articular routes); methotrexate, lefunomide; hydroxychloroquine, d-penicillamine, auranofin or other parenteral or oral gold preparations.

The present invention still further relates to the combination of a compound of the invention together with a leukotriene biosynthesis inhibitor, 5-lipoxygenase (5-LO) inhibitor or 5-lipoxygenase activating protein (FLAP) antagonist such as; zileuton; ABT-761; fenleuton; tepoxalin; Abbott-79175; Abbott-85761; N-(5-substituted)-thiophene-2-alkylsulfonamides; 2,6-di-tert-butylphenol hydrazones; methoxytetrahydropyrans such as Zeneca ZD-2138; the compound SB-210661; pyridinyl-substituted 2-cyanonaphthalene compounds such as L-739,010; 2-cyanoquinoline compounds such as L-746,530; indole and quinoline compounds such as MK-591, MK-886, and BAY x 1005.

The present invention still further relates to the combination of a compound of the invention together with a receptor antagonist for leukotrienes( LT)B4, LTC4, LTD4, and LTE4. selected from the group consisting of the phenothiazin-3-1s such as L-651,392; amidino compounds such as CGS-25019c; benzoxalamines such as ontazolast; benzenecarboximidamides such as BIIL 284/260; and compounds such as zafirlukast, ablukast, montelukast, pranlukast, verlukast (MK-679), RG-12525, Ro-245913, iralukast (CGP 45715A), and BAY x 7195.

The present invention still further relates to the combination of a compound of the invention together with a phosphodiesterase (PDE) inhibitor such as the methylxanthanines including theophylline and aminophylline; and selective PDE isoenzyme inhibitors including PDE4 inhibitors and inhibitors of the isoform PDE4D, and inhibitors of PDE5.

The present invention still further relates to the combination of a compound of the invention together with histamine type 1 receptor antagonists such as cetirizine, loratadine, desloratadine, fexofenadine, acrivastine, terfenadine, astemizole, azelastine, levocabastine, chlorpheniramine, promethazine, cyclizine, and mizolastine applied orally, topically or parenterally.

The present invention still further relates to the combination of a compound of the invention together with a gastroprotective histamine type 2 receptor antagonist.

The present invention still further relates to the combination of a compound of the invention with antagonists of the histamine type 4 receptor.

The present invention still further relates to the combination of a compound of the invention together with an alpha-1/alpha-2 adrenoceptor agonist vasoconstrictor sympathomimetic agent, such as propylhexedrine, phenylephrine, phenylpropanolamine, ephedrine, pseudoephedrine, naphazoline hydrochloride, oxymetazoline hydrochloride, tetrahydrozoline hydrochloride, xylometazoline hydrochloride, tramazoline hydrochloride, and ethylnorepinephrine hydrochloride.

The present invention still further relates to the combination of a compound of the invention together with anticholinergic agents including muscarinic receptor (M1, M2, and M3) antagonists such as atropine, hyoscine, glycpyrrrolate, ipratropium bromide; tiotropium bromide; oxitropium bromide; pirenzepine; and telenzepine.

The present invention still further relates to the combination of a compound of the invention together with a beta-adrenoceptor agonist (including beta receptor subtypes 1-4) such as isoprenaline, salbutamol, formoterol, salmeterol, terbutaline, orciprenaline, bitolterol mesylate, and pirbuterol.

The present invention still further relates to the combination of a compound of the invention together with a chromone, including sodium cromoglycate and nedocromil sodium.

The present invention still further relates to the combination of a compound of the invention together with an insulin-like growth factor type I (IGF-1) mimetic.

The present invention still further relates to the combination of a compound of the invention together with an inhaled glucocorticoid, such as flunisolide, triamcinolone acetonide, beclomethasone dipropionate, budesonide, fluticasone propionate, ciclesonide, and mometasone furoate.

The present invention still further relates to the combination of a compound of the invention together with an inhibitor of matrix metalloproteases (MMPs), i.e., the stromelysins, the collagenases, and the gelatinases, as well as aggrecanase; especially collagenase-1 (MMP-1), collagenase-2 (MMP-δ), collagenase-3 (MMP-13), stromelysin-1 (MMP-3), stromelysin-2 (MMP-10), and stromelysin-3 (MMP-11) and MMP-9 and MMP-12.

The present invention still further relates to the combination of a compound of the invention together with modulators of chemokine receptor function such as antagonists of CCR1, CCR2, CCR2A, CCR2B, CCR3, CCR4, CCR5, CCR6, CCR7, CCR8, CCR9, CCR10 and CCR11 (for the C-C family); CXCR1, CXCR2, CXCR3, CXCR4 and CXCR5 (for the C-X-C family) and CX₃CR1 for the C-X₃-C family.

The present invention still further relates to the combination of a compound of the invention together with a cytokine or modulator of cytokine function, including alpha-, beta-, and gamma-interferon; interleukins (IL) including IL1 to 15, and interleukin antagonists or inhibitors, including agents which act on cytokine signalling pathways.

The present invention still further relates to the combination of a compound of the invention together with an immunoglobulin (Ig) or Ig preparation or an antagonist or antibody modulating Ig function such as anti-IgE (omalizumab).

The present invention still further relates to the combination of a compound of the invention together with other systemic or topically-applied anti-inflammatory agents including thalidomide and derivatives, retinoids, dithranol, and calcipotriol.

The present invention still further relates to the combination of a compound of the invention together with an antibacterial agent including penicillin derivatives, tetracyclines, macrolides, beta-lactams, flouroquinolones, and inhaled aminoglycosides; and antiviral agents including acyclovir, famciclovir, valaciclovir, ganciclovir, cidofovir; amantadine, rimantadine; ribavirin; zanamavir and oseltamavir; protease inhibitors such as indinavir, nelfinavir, ritonavir, and saquinavir; nucleoside reverse transcriptase inhibitors such as didanosine, lamivudine, stavudine, zalcitabine, zidovudine; non-nucleoside reverse transcriptase inhibitors such as nevirapine, efavirenz.

The present invention still further relates to the combination of a compound of the invention together with cardiovascular agents such as calcium channel blockers, beta-adrenoceptor blockers, angiotensin-converting enzyme (ACE) inhibitors, angiotensin-2 receptor antagonists; lipid lowering agents such as statins, and fibrates; modulators of blood cell morphology such as pentoxyfylline; thrombolytics, and anticoagulants including platelet aggregation inhibitors.

The present invention still further relates to the combination of a compound of the invention together with CNS agents such as antidepressants (such as sertraline), anti-Parkinsonian drugs (such as deprenyl, L-dopa, Requip, Mirapex, MAOB inhibitors such as selegine and rasagiline, comP inhibitors such as Tasmar, A-2 inhibitors, dopamine reuptake inhibitors, NMDA antagonists, nicotine agonists, dopamine agonists and inhibitors of neuronal nitric oxide synthase), and anti-Alzheimer's drugs such as donepezil, tacrine, COX-2 inhibitors, propentofylline or metrifonate.

The present invention still further relates to the combination of a compound of the invention together with agents for the treatment of acute and chronic pain, including centrally and peripherally-acting analgesics such as opioid analogues and derivatives, carbamazepine, phenytoin, sodium valproate, amitryptiline and other antidepressant agents, and non-steroidal anti-inflammatory agents.

The present invention still further relates to the combination of a compound of the invention together with parenterally or topically-applied local anaesthetic agents such as lignocaine.

The present invention still further relates to the combination of a compound of the invention together with (i) tryptase inhibitors; (ii) platelet activating factor (PAF) antagonists; (iii) interleukin converting enzyme (ICE) inhibitors; (iv) IMPDH inhibitors; (v) adhesion molecule inhibitors including VLA-4 antagonists; (vi) cathepsins; (vii) MAP kinase inhibitors; (viii) glucose-6 phosphate dehydrogenase inhibitors; (ix) kinin-B.sub1. - and B.sub2. -receptor antagonists; (x) anti-gout agents, e.g., colchicine; (xi) xanthine oxidase inhibitors, e.g., allopurinol; (xii) uricosuric agents, e.g., probenecid, sulfinpyrazone, and benzbromarone; (xiii) growth hormone secretagogues; (xiv) transforming growth factor (TGFβ); (xv) platelet-derived growth factor (PDGF); (xvi) fibroblast growth factor, e.g., basic fibroblast growth factor (bFGF); (xvii) granulocyte macrophage colony stimulating factor (GM-CSF); (xviii) capsaicin cream; (xix) Tachykinin NK.sub1. and NK.sub3. receptor antagonists selected from the group consisting of NKP-608C; SB-233412 (talnetant); and-4418; (xx) elastase inhibitors selected from the group consisting of UT-77 and ZD-0892; (xxi) TNF□ converting enzyme inhibitors (TACE); (xxii) induced nitric oxide synthase inhibitors (iNOS) or (xxiii) chemoattractant receptor-homologous molecule expressed on TH2 cells, (CRTH2 antagonists) (xxiv) inhibitors of P38

The compounds of the present invention may also be used in combination with anti-osteoporosis agents including hormonal agents such as raloxifene, and biphosphonates such as alendronate.

The compounds of the invention may also be used in combination with existing therapeutic agents for the treatment of osteoarthritis. Suitable agents to be used in combination include standard non-steroidal anti-inflammatory agents (hereinafter NSAIDs) such as piroxicam, diclofenac, propionic acids such as naproxen, flubiprofen, fenoprofen, ketoprofen and ibuprofen, fenamates such as mefenamic acid, indomethacin, sulindac, apazone, pyrazolones such as phenylbutazone, salicylates such as aspirin, COX-2 inhibitors such as celecoxib, valdecoxib, rofecoxib and etoricoxib, analgesics, and intra-articular therapies such as corticosteroids and hyaluronic acid derivatives, and nutritional supplements such as glucosamine.

The compounds of the invention can also be used in combination with existing therapeutic agents for the treatment of cancer. Suitable agents to be used in combination include:
(i) antiproliferative/antineoplastic drugs and combinations thereof, as used in medical oncology, such as alkylating agents (for example cis-platin, carboplatin, cyclophosphamide, nitrogen mustard, melphalan, chlorambucil, busulphan and nitrosoureas); antimetabolites (for example antifolates such as fluoropyrimidines like 5-fluorouracil and tegafur, raltitrexed, methotrexate, cytosine arabinoside, hydroxyurea, gemcitabine and paclitaxel; antitumour antibiotics (for example anthracyclines like adriamycin, bleomycin, doxorubicin, daunomycin, epirubicin, idarubicin, mitomycin-C, dactinomycin and mithramycin); antimitotic agents (for example vinca alkaloids like vincristine, vinblastine, vindesine and vinorelbine and taxoids like taxol and taxotere); and topoisomerase inhibitors (for example epipodophyllotoxins like etoposide and teniposide, amsacrine, topotecan and camptothecins);
(ii) cytostatic agents such as antioestrogens (for example tamoxifen, toremifene, raloxifene, droloxifene and iodoxyfene), oestrogen receptor down regulators (for example fulvestrant), antiandrogens (for example bicalutamide, flutamide, nilutamide and cyproterone acetate), LHRH antagonists or LHRH agonists (for example goserelin, leuprorelin and buserelin), progestogens (for example megestrol acetate), aromatase inhibitors (for example as anastrozole, letrozole, vorazole and exemestane) and inhibitors of 5α-reductase such as finasteride;
(iii) Agents which inhibit cancer cell invasion (for example metalloproteinase inhibitors like marimastat and inhibitors of urokinase plasminogen activator receptor function);
(iv) inhibitors of growth factor function, for example such inhibitors include growth factor antibodies, growth factor receptor antibodies (for example the anti-erbb2 antibody trastuzumab and the anti-erbb1 antibody cetuximab [C225]) , farnesyl transferase inhibitors, tyrosine kinase inhibitors and serine/threonine kinase inhibitors, for example inhibitors of the epidermal growth factor family (for example EGFR family tyrosine kinase inhibitors such as N-(3-chloro-4-fluorophenyl)-7-methoxy-6-(3-morpholinopropoxy)quinazolin-4-amine (gefitinib, AZD1839), N-(3-ethynylphenyl)-6,7-bis(2-methoxyethoxy)quinazolin-4-amine (erlotinib, OSI-774) and 6-acrylamido-N-(3-chloro-4-fluorophenyl)-7-(3-morpholinopropoxy)quinazolin-4-amine (CI 1033)), for example inhibitors of the platelet-derived growth factor family and for example inhibitors of the hepatocyte growth factor family;
(v) antiangiogenic agents such as those which inhibit the effects of vascular endothelial growth factor, (for example the anti-vascular endothelial cell growth factor antibody bevacizumab, compounds such as those disclosed in International Patent Applications WO 97/22596, WO 97/30035, WO 97/32856 and WO 98/13354) and compounds that work by other mechanisms (for example linomide, inhibitors of integrin αvβ3 function and angiostatin);
(vi) vascular damaging agents such as combretastatin A4 and compounds disclosed in International Patent Applications WO 99/02166, VO00/40529, WO 00/41669, WO01/92224, WO02/04434 and WO02/08213;
(vii) antisense therapies, for Example those which are directed to the targets listed above, such as ISIS 2503, an anti-ras antisense;
(viii) gene therapy approaches, including for example approaches to replace aberrant genes such as aberrant p53 or aberrant BRCA1 or BRCA2, GDEPT (gene-directed enzyme pro-drug therapy) approaches such as those using cytosine deaminase, thymidine kinase or a bacterial nitroreductase enzyme and approaches to increase patient tolerance to chemotherapy or radiotherapy such as multi-drug resistance gene therapy; and
(ix) immunotherapy approaches, including for example ex-vivo aud in-vivo approaches to increase the immunogenicity of patient tumour cells, such as transfection with cytokines such as interleukin 2, interleukin 4 or granulocyte-macrophage colony stimulating factor, approaches to decrease T-cell anergy, approaches using transfected immune cells such as cytokine-transfected dendritic cells, approaches using cytokine-transfected tumour cell lines and approaches using anti-idiotypic antibodies.

In a still further aspect, the present invention provides the use of a compound of formula (I), or a pharmaceutically acceptable salt or solvate thereof, as hereinbefore defined in the manufacture of a medicament for the treatment of human diseases or conditions in which modulation of CRrn12 preceptor activity is beneficial.

In the context of the present specification, the term "therapy" also includes "prophylaxis" unless there are specific indications to the contrary. The terms "therapeutic" and "therapeutically" should he construed accordingly.

The invention still further provides a method of treating diseases mediated by PGD2 or its metabolites wherein the prostanoid binds to its receptor (especially CRTb2) receptor, which comprises administering to a patient a therapeutically effective amount of a compound of formula (I), or a pharmaceutically acceptable salt or solvate thereof, as hereinbefore defined.

The invention also provides a method of treating an inflammatory disease, especially psoriasis, in a patient suffering from, or at risk of, said disease, which comprises psoriasis, in a patient suffering from, effective amount of a compound of formula (I), or a pharmaceutically acceptable salt or solvante thereof, as hereinbefore defined.

For the above-mentioned therapeutic uses the dosage administered will, of course, vary with the compound employed, the mode of administration, the treatment desired and the disorder indicated.

For the above-mentioned therapeutic uses the dosage administered will, of course, vary with the compound employed, the mode of administration, the treatment desired and the disorder indicated.

The compound of formula (I) and pharmaceutically acceptable salts and solvates thereof may be used on their own but will generally be administered in the form of a pharmaceutical composition in which the formula (I) compound/salt/solvate (active ingredient) is in association with a pharmaceutically acceptable adjuvant, diluent or carrier. Depending on the mode of administration, the pharmaceutical composition will preferably comprise from 0.05 to 99 %w (per cent by weight), more preferably from 0-05 to 80 %w, still more preferably from 0.10 to 70 %w, and even more preferably from 0.10 to 50 %w, of active ingredient, all percentages by weight being based on total composition.

The present invention also provides a pharmaceutical composition comprising a compound of formula (I), or a pharmaceutically acceptable salt or solvate thereof, as herein before defined, in association with a pharmaceutically acceptable adjuvant, diluent or carrier.

The pharmaceutical compositions may be administered topically (e.g. to the lung and/or airways or to the skin) in the form of solutions, suspensions, heptafluoroalkane aerosols and dry powder formulations; or systemically, e.g. by oral administration in the form of tablets, capsules, syrups, powders or granules, or by parenteral administration in the form of solutions or suspensions, or by subcutaneous administration or by rectal administration in the form of suppositories or transdermally. Preferably the compound of the invention is administered orally.

The invention will now be illustrated by the following examples in which, unless stated otherwise:
(i) when given, ¹H NMR data is quoted in the form of delta values for major diagnostic protons, given in parts per million (ppm) relative to tetramethylsilane (TMS) as an internal standard;
(ii) mass spectra (MS): generally only ions which indicate the parent mass are reported, and unless otherwise stated the mass ion quoted is the positive mass ion - (M+H)⁺;
(iii) the title compounds of the examples and methods were named using the ACD/name and ACD/name batch (version 6.0) from Advanced Chemical Development Inc, Canada;
(iv) unless stated otherwise, reverse phase HPLC was conducted using a Symmetry, NovaPak or Ex-Terra reverse phase silica column;
(v) solvents were dried with MgSO₄ or Na₂SO₄
(vi) the following abbreviations are used:

| | |
|---|---|
| EtOAc | Ethylacetate |
| DCM | Dichloromethane |
| NMP | N-methylpyrrolidine |
| DMF | N,N-dimethylformamide |
| THF | tetrahydrofuran |
| mcpba | 3-chloroperoxybenzoic acid (Aldrich 77% max) |
| Pd(dppf)Cl₂ | [1,1'-Bis(diphenylphosphino)ferrocene]dichloropalladium(II), |
| | complex with dichloromethane |
| RT | room temperature |

### Example 1

### [(5-Methylbiphenyl-2-yl)oxy]acetic acid

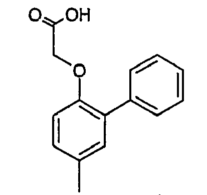

(i) tert-Butyl (2-bromo-4-nitrophenoxy)acetate
   tert-Butyl bromoacetate (3.06ml) was added to a stirred mixture of 2-bromo-4-nitrophenol (4g) and potassium carbonate (2.62g) in DMF (40ml) at RT. After 18h the reaction was partitioned between diethylether and water, the organics separated, dried and evaporated under reduced pressure. The residue was triturated with iso-hexane and filtered. Yield 5.6g ¹H NMR CDCl₃: δ 8.49 (1H, d) ; 8.21-8.16 (1H, m) ; 6.82 (1H, d) ; 4.71 (2H, s) ; 1.49 (9H, s)
(ii) tert-Butyl [(5-nitrobiphenyl-2-yl)oxy]acetate
   A mixture of the product from step (i) (5.6g), benzeneboronic acid (2.04g), cesium fluoride (5.1g) and Pd(dppf)Cl₂ (0.6g) in dioxane (60ml) was heated under reflux for 4h. After cooling the mixture was partitioned between diethylether and water. The organics were separated, dried and evaporated under reduced pressure.
   MS: APCI (+ve): 272 (M+1- ^{t}Bu)
(iii) tert-Butyl [(5-aminobiphenyl-2-yl)oxy]acetate
   Iron powder (5g) was added to a solution of the product from step (ii) in acetic acid (100ml) and stirred at RT for 16h. The mixture was filtered through celite and evaporated under reduced pressure. The resulting oil was made basic with aqueous sodium hydroxide solution then extracted with EtOAc. The organics were dried, evaporated under reduced pressure and the residue purified by chromatography on silica eluting with 8:1 DCM/EtOAc. Yield 3.74g
   MS: APCI (-ve): 272 (M-1-^{t}Bu)
(iv) tert-Butyl [(5-bromobiphenyl-2-yl)oxy]acetate
   Copper (II) bromide (2.67 g) was added to a mixture of the product from step (iii) (3g) and isoamylnitrite (2ml) in acetonitrile (40ml) and heated at 65°C for 2h. The solvent was evaporated under reduced pressure and the residue purified by chromatography on silica eluting with 5:1 isohexane/diethylether. Yield 2.33g
   MS: APCI (-ve): 306/7 (M-1-^{t}Bu)
(v) tert-Butyl [(5-methylbiphenyl-2-yl)oxy] acetate
   A mixture of the product from step (iv) (0.5g), methylzinc chloride (3.44ml, 2M in THF) and Pd(dppf)Cl₂ (0.1g) in THF (10ml) was heated at 90°C for 4h. After cooling the mixture was partitioned between diethylether and water, the organics separated, dried and evaporated under reduced pressure. The residue was purified by chromatography on silica eluting with 4:1 isohexane/diethylether. Yield 0.43g
   MS: APCI (-ve): 241 (M-1-^{t}Bu)
(vi) [(5-Methylbiphenyl-2-yl)oxy]acetic acid
   A solution of the product from step (v) (0.43g) and trifluoroacetic acid (10ml) in DCM (10ml) was stirred at RT for 1h then evaporated under reduced pressure. The residue was purified by chromatography on silica eluting with 1:1 DCM/EtOAc + 1% AcOH then by RPHPLC. Yield 0.03g
   ¹H NMR DMSO-d6: δ 7.56-6.85 (8H, m) ; 4.64 (2H, s) ; 2.27 (3H, s)
   MS: APCI (-ve): 241 (M-1)

### Example 2

### {[5-Ethyl-4'-(methylsulfonyl)biphenyl-2-yl]oxy}acetic acid

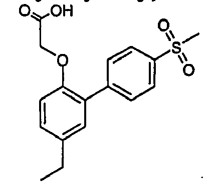

(i) tert-Butyl (4-ethyl-2-iodophenoxy)acetate
   Sodium iodide (4.41g) then chloramine-T (8.29g) was added to a stirred solution of 4-ethylphenol (3g) at 0°C then allowed to warm to RT. After 1h the mixture was diluted with 2M hydrochloric acid and extracted with diethylether. The organic layer was washed with aqueous sodium thiosulphate solution, dried and evaporated under reduced pressure. The residue was dissolved in DMF (30ml) then tert-butyl bromoacetate (3.9ml) and potassium carbonate (3.31g) added and stirred at RT overnight. The mixture was partitioned between water and diethylether, the organics dried and evaporated under reduced pressure. The residue was purified by chromatography on silica eluting with 20% diethylether/isohexane. Yield 8.6g
   MS: APCI (-ve): 305 (M-1-^{t}Bu)
(ii) tert-Butyl {[5-ethyl-4'-(methylthio)biphenyl-2-yl]oxy} acetate
   The subtitle compound was prepared by the method of example 1 step (ii) using the product from step (i) and 4-(methylthio)benzeneboronic acid. Yield 1.2g
   MS: APCI (-ve): 301 (M-1-^{t}Bu)
(iii) {[5-Ethyl-4'-(methylsulfonyl)biphenyl-2-yl]oxy} acetic acid
   Mcpba (1.44g) was added to a stirred solution of the product from step (ii) (1.2g) in DCM (10ml) at RT. After 16h, the mixture was partitioned between DCM and aqueous sodium metabisulphite solution, the organics separated, washed with aqueous sodium hydrogencarbonate solution, water, dried and evaporated under reduced pressure. The residue was dissolved in trifluoroacetic acid (10ml) and DCM (10ml), stirred at RT for 2h then evaporated under reduced pressure. The residue was purified by RPHPLC. Yield 0.035g
   ¹H NMR DMSO-d6: δ 7.95-6.94 (7H, m) ; 4.71 (2H, s) ; 3.25 (3H, s) ; 2.62-2.57 (2H, q) ; 1.20-1.17 (3H, t)
   MS: APCI (-ve): 333 (M-1)

### Example 3

### {[4'-(Ethylsulfonyl)-5-methoxybiphenyl-2-yl]oxy}acetic acid

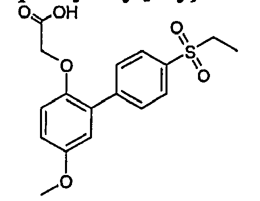

(i) tert-Butyl (2-bromo-4-methoxyphenoxy)acetate
   The subtitle compound was prepared by the method of example 1 step (i) using 2-bromo-4-methoxyphenol. Yield 1.9g
   MS: APCI (-ve): 251 (M-1-^{t}Bu)
(ii) tert-Butyl {[4'-(ethylthio)-5-methoxybiphenyl-2-yl]oxy} acetate
   The subtitle compound was prepared by the method of example 1 step (ii) using the product from step (i) and 4-(ethylthio)benzeneboronic acid. Yield 1.15g
   MS: APCI (-ve): 317 (M-1-^{t}Bu)
(iii) {[4'-(Ethylsulfonyl)-5-methoxybiphenyl-2-yl]oxy}acetic acid
   The title compound was prepared by the method of example 2 step (iii) using the product from step (ii). Yield 0.12g
   ¹H NMR DMSO-d6: δ 7.92-7.85 (4H, m) ; 7.01-6.92 (3H, m) ; 4.68-4.66 (2H, s) ; 3.76 (3H, s) ; 3.37-3.29 (2H, m) ; 1.17-1.12 (3H, t)
   MS: APCI (-ve): 349 (M-1)

### Example 4

### [[4-Chloro-4'-(ethylsulfonyl)-2',5-dimethyl[1,1'-biphenyl]-2-yl]oxy]-acetic acid

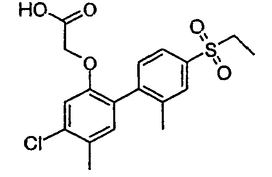

(i) (2-Bromo-5-chloro-4-methylphenoxy)-acetic acid, 1,1-dimethylethyl ester
   The subtitle compound was prepared by the method of example 1 step (i) using 6-bromo-4-chloro-m-cresol. Yield 0.8g
   ¹H NMR CDCl₃: δ 7.52 (1H, s) ; 6.65 (1H, s) ; 4.60 (2H, s) ; 2.30 (3H, s) ; 1.46 (9H, s)
(ii) 4-Bromo-3-methylphenyl ethyl sulfide
   Bromine (2.2ml) was added to a solution of 1-(ethylthio)-3-methylbenzene (6.6g) in acetic acid (20ml) at 0°C. The mixture was stirred at RT for 2h then the solvent removed under reduced pressure. The residue was purified by chromatography on silica eluting with DCM. Yield 6.6g
   MS: APCI (+ve): 247/9 (M+1)
(iii) [4-(Ethylthio)-2-methylphenyl]-boronic acid
   A 100ml portion of a solution of the product from step (ii) (120.7g) in THF (500ml) was added to a stirred mixture of magnesium turnings (13.4g) in THF (100ml). Dibromoethane (0.2ml) was added, and the mixture gently refluxed on initiation. The remaining bromide solution was added dropwise maintaining the reaction at reflux. After addition the mixture was allowed to cool to RT then cannulated into a stirred solution of trimethylborate (112ml) in THF (200ml) at 0°C. The mixture was warmed to RT, stirred for 2h then quenched with 2M hydrochloric acid (300ml). After stirring at RT for 18h the THF was removed under reduced pressure and the mixture extracted with diethylether. The organics were separated, washed with water, dried and evaporated under reduced pressure. The residue was triturated with diethylether/isohexane and filtered. Yield 53.02g
   ¹H NMR CDCl₃: δ 8.08 (1H, d) ; 7.18 (1H, d) ; 7.15 (1H, s) ; 3.04 (2H, q) ; 2.76 (3H, s) ; 1.38 (3H, t)
(iv) [[4-Chloro-4'-(ethylsulfonyl)-2',5-dimethyl[1,1'-biphenyl]-2-yl]oxy]-acetic acid A mixture of the product from step (i) (0.38g), product from step (iii) (0.32g), cesium fluoride (0.35g) and Pd(dppf)Cl₂ (0.12g) in dioxane (5ml) was heated at 90°C for 24h. The solvent was evaporated under reduced pressure and the residue passed through a plug of silica eluting with isohexane then 10% ethylacetate/isohexane. The resulting oil was dissolved in DCM (10ml) then trifluoroacetic acid (3ml) added and the mixture stirred for 1h at RT the evaporated under reduced pressure. The residue was dissolved in water (10ml) and acetonitrile (10ml) then oxone (2.5g) added and stirred at RT. After 2h the acetonitrile was removed under reduced pressure and the mixture extracted with ethylacetate. The organic layer was extracted with aqueous sodium hydroxide solution, then the aqeous layer acidified with 2M hydrochloric acid and extracted with ethylacetate. The last ethylacetate extracts were dried and evaporated under reduced pressure. The residue was purified by RPHPLC eluting with acetonitrile/aqueous trifluoroacetic acid. Yield 0.023g
   ¹H NMR CDCl₃: δ 8.01 (1H, s); 7.77 (1H, d) ; 7.42 (1H, d) ; 7.18 (1H, s) ; 7.00 (1H, s); 3.35 (2H, q) ; 2.50 (3H, s) ; 2.24 (3H, s) ; 1.13 (3H, t)
   MS: ESI (-ve): 381 (M-1)

### Example 5

### [[4'-(Ethylsulfonyl)-2',5-dimethyl[1,1'-biphenyl]-2-yl]oxy]-acetic acid

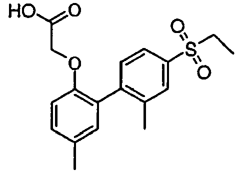

The title compound was prepared by the method of example 4, yield 0.066g.
¹H NMR CDCl₃: δ 7.76 (1H, s) ; 7.71 (1H, dd); 7.41 (1H, d) ; 7.20 (1H, dd) ; 6.96 (1H, s) ; 6.84 (1H, d) ; 4.60 (2H, s) ; 3.30 (2H, q) ; 2.27 (3H, s) ; 2.06 (3H, s) ; 1.12 (3H, t)
MS: APCI (-ve): 347 (M-1)

### Example 6

### 2-[[3'-Cyano-5-methyl[1,1'-biphenyl]-2-yl]oxy]-(2S)-propanoic acid

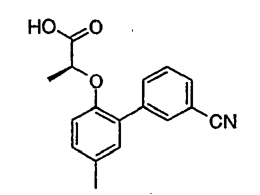

i) 5-Methyl-2-methoxyphenylboronic acid
   The subtitle compound was prepared by the method of example 4 step (iii) using 4-methyl-2-bromoanisole (7.0ml). Yield 2.6g
   ¹H NMR DMSO: δ 7.64 (2H, s); 7.38 (1H, s); 7.18 (1H, d); 6.86 (1H, d); 3.77 (3H, s); 2.22 (3H, s)
ii) 2'-Methoxy-5'-methyl-[1,1'-biphenyl]-3-carbonitrile
   A mixture of the product from step (i) (0.8g), 3-bromobenzonitrile (0.877g), 2M aqueous sodium carbonate (3.0ml) and tetrakistriphenylphosphine palladium (0) (0.2g) in toluene (12ml) and ethanol (3ml) was heated at reflux for 48h. The mixture was partitioned between EtOAc and water, the organics separated, dried, and evaporated under reduced pressure. The residue was purified by chromatography on silica eluting with 10% EtOAc/isohexane. Yield 0.92g
   ¹H NMR DMSO: δ 7.9 (1H, d) ; 7.8 (2H, m) ; 7.61 (1H, t) ; 7.16-7.22 (2H, m) ; 7.04 (1H, d) ; 3.75 (3H, s) ; 2.29 (3H, s)
iii) 2'-Hydroxy-5'-methyl-[1,1'-biphenyl]-3-carbonitrile
   A solution of boron tribromide(1M inDCM) (8ml) was added to a solution of the product from step (ii) (0.92g) in DCM (20ml) at 0°C and stirred for 2h at 0°C and at RT for 20h. The reaction was quenched with ice, extracted with DCM, washed with brine, dried (MgSO₄) and evaporated to give the subtitle compound. Yield 0.77g
   ¹H NMR DMSO: δ 9.52 (1H, s) ; 7.95 (1H, d) ; 7.89 (1H, dt) ; 7.75 (1H, dt) ; 7.6 (1H, t) ; 7.14 (1H, d) ; 7.02 (1H, dd) ; 6.86 (1H, d) ; 2.25 (3H, s)
iv) 2-[[3'-Cyano-5-methyl[1,1'-biphenyl]-2-yl]oxy]-(2S)-propanoic acid, 1,1-dimethylethyl ester
   Diethyl azodicarboxylate (0.3ml) was added to a stirred solution of the product from step (iii) (0.3g), tert-butyl-R-lactate (0.21g) and triphenylphosphine (0.375g) in THF (10ml) at 0°C. Stirred for 2h, absorbed onto silica and purified by chromatography on silica eluting with 10% EtOAc/isohexane to give the subtitle compound. Yield 0.386g
   ¹H NMR DMSO: δ 8.04 (1H, s) ; 7.95 (1H, d) ; 7.78 (1H, d) ; 7.63 (1H, t) ; 7.22 (1H, d) ; 7.15 (1H, dd) ; 6.86 (1H, d) ; 4.84 (1H, q) ; 2.28 (3H, s) ; 1.39 (3H, d) ; 1.38 (9H, s)
v) 2-[[3'-Cyano-5-methyl[1,1'-biphenyl]-2-yl]oxy]-(2S)-propanoic acid
   The title compound was prepared by the method of example 1 step (vi) using the product from step (iv). Yield 0.075g
   ¹H NMR DMSO: δ 8.23 (1H, t); 8.06 (1H, dt) ; 7.75 (1H, dt) ; 7.6 (1H, t); 7.17 (1H, d) ; 7.08 (1H, dd) ; 6.85 (1H, d) ; 4.61 (1H, q) ; 2.27 (3H, s) ; 1.35 (3H, d)
   MS: APCI (-ve): 280

### Example 7

### 2-[[2'-Fluoro-5'-cyano-5-methyl[1,1'-biphenyl]-2-yl]oxy]-(2S)-propanoic acid

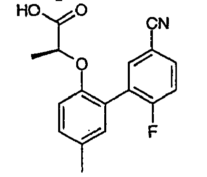

The title compound was prepared by the method of example 4, using 3-bromo-4-fluorobenzonitrile.
¹H NMR DMSO: δ 8.21 (1H, dd) ; 7.89 (1H, ddd) ; 7.5 (1H, t) ; 7.09-7.16 (2H, m) ; 6.86 (1H, d) ; 4.58 (1H, q) ; 2.27 (3H, s) ; 1.3 (3H, d)
MS: APCI (-ve): 298

### Pharmacological Data

### Ligand Binding Assay

[³H]PGD₂ was purchased from Perkin Elmer Life Sciences with a specific activity of 100-210Ci/mmol. All other chemicals were of analytical grade.

HEK cells expressing rhCRTh2 / Gα16 were routinely maintained in DMEM containing 10% Foetal Bovine Serum (HyClone), 1mg/ml geneticin, 2mM L-glutamine and 1% non-essential amino acids. For the preparation of membranes, the adherent transfected HEKcells were grown to confluence in two layer tissue culture factories (Fisher, catalogue number TKT-170-070E). Maximal levels of receptor expression were induced by addition of 500mM sodium butyrate for the last 18 hours of culture. The adherent cells were washed once with phosphate buffered saline (PBS, 50ml per cell factory) and detached by the addition of 50ml per cell factory of ice-cold membrane homogenisation buffer [20mM HEPES (pH 7.4), 0.1mM dithiothreitol, 1mM EDTA, 0.1mM phenyl methyl sulphonyl fluoride and 100µg/ml bacitracin]. Cells were pelleted by centrifugation at 220xg for 10 minutes at 4°C, re-suspended in half the original volume of fresh membrane homogenisation buffer and disrupted using a Polytron homogeniser for 2 x 20 second bursts keeping the tube in ice at all times. Unbroken cells were removed by centrifugation at 220xg for 10 minutes at 4°C and the membrane fraction pelleted by centrifugation at 90000xg for 30 minutes at 4°C. The final pellet was re-suspended in 4 ml of membrane homogenisation buffer per cell factory used and the protein content determined.
Membranes were stored at -80°C in suitable aliquots.

All assays were performed in Corning clear bottomed, white 96-well NBS plates (Fisher). Prior to assay, the HEK cells membranes containing CRTh2 were coated onto SPA PVT WGA beads (Amersham). For coating membranes were incubated with beads at typically 25µg membrane protein per mg beads at 4°C with constant agitation overnight. (The optimum coating concentrations were determined for each batch of membranes) The beads were pelleted by centrifugation (800xg for 7minutes at 4°C), washed once with assay buffer (50mM HEPES pH 7.4 containing 5mM magnesium chloride) and finally re-suspended in assay buffer at a bead concentration of 10mg/ml.

Each assay contained 20µl of 6.25nM [³H]PGD₂, 20µl membrane saturated SPA beads both in assay buffer and 10µl of compound solution or 13,14-dihydro-15-keto prostaglandin D₂ (DK-PGD₂, for determination of non-specific binding, Cayman chemical company). Compounds and DK-PGD₂ were dissolved in DMSO and diluted in the same solvent to 100x the required final concentration. Assay buffer was added to give a final concentration of 10% DMSO (compounds were now at 10x the required final concentration) and this was the solution added to the assay plate. The assay plate was incubated at room temperature for 2 hours and counted on a Wallac Microbeta liquid scintillation counter (1 minute per well).

Compounds of formula (I) have an IC₅₀ value of less than (<) 10µM.
Specifically, example 2 has a pIC₅₀ = 7.1 and example 3 has a pIC₅₀ = 6.6.

## Claims

1. A compound of formula (I) or a pharmaceutically acceptable salt thereof: 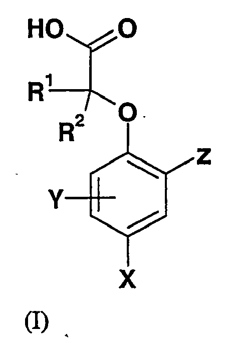 in which:
X is C₁₋₆alkyl or OR⁶;
Y is selected from hydrogen, halogen, CN, nitro, SO₂R³, OR⁴, SR⁴, SOR³, SO₂NR⁴R⁵, CONR⁴R⁵, NR⁴R⁵, NR⁶SO₂R³, NR⁶CO₂R⁶, NR⁶COR³, C₂-C₆ alkenyl, C₂-C₆ alkynyl,
C₃-C₇ cycloalkyl or C₁₋₆alkyl, the latter four groups being optionally substituted by one or more substituents independently selected from halogen, OR⁶ and NR⁶R⁷, S(O)ₙR⁶ where n is 0, 1 or 2;
Z is aryl or a ring A, where A is a six membered heterocyclic aromatic ring containing one or more nitrogen atoms or may be a 6,6 or 6,5 fused bicycle containing one or more O, N, S atoms, the aryl or A rings all being optionally substituted by one or more substituents independently selected from from hydrogen, halogen, CN, OH, SH, nitro, COR⁹, CO₂R⁶, SO₂R⁹, OR⁹, SR⁹, SOR⁹, SO₂NR¹⁰R¹¹, CONR¹⁰R¹¹, NR¹⁰R¹¹, NHSO₂R⁹, NR⁹SO₂R⁹, NR⁶CO₂R⁶, NHCOR⁹, NR⁹COR⁹, NR⁶CONR⁴R⁵, NR⁶SO₂NR⁴R⁵, aryl, heteroaryl,
C₂-C₆ alkenyl, C₂-C₆ alkynyl, C₃-C₇ cycloalkyl or C₁₋₆alkyl, the latter four groups being optionally substituted by one or more substituents independently selected from halogen,
C₃-C₇ cycloalkyl, OR⁶, NR⁶R⁷, S(O)ₙR⁶ (where n is 0, 1 or 2), CONR⁶R⁷, NR⁶COR⁷, SO₂NR⁶R⁷ and NR⁶SO₂R⁷.
R¹ and R² independently represent a hydrogen atom, halogen, C₂-C₆ alkenyl, C₂-C₆ alkynyl, C₃-C₇ cycloalkyl or a C₁₋₆alkyl group, the latter four groups being optionally substituted by one or more substituents independently selected from halogen,
C₃-C₇ cycloalkyl, NR⁶R⁷, OR⁶, S(O)ₙR⁶ (where n is 0, 1 or 2);
or
R¹ and R² together can form a 3-8 membered ring optionally containing one or more atoms selected from O, S, NR⁶ and itself optionally substituted by one or more C₁-C₃ alkyl or halogen;
R³ represents C₃-C₇ cycloalkyl or C₁₋₆alkyl which may be optionally substituted by one or more substituents independently selected from halogen, C₃-C₇ cycloalkyl, OR⁶ and NR⁶R⁷, S(O)ₙR⁶ (where n = 0,1 or 2), CONR⁶R⁷, NR⁶COR⁷, SO₂NR⁶R⁷ and NR⁶SO₂R⁷;
R⁴ and R⁵ independently represent hydrogen, C₃-C₇ cycloalkyl or C₁₋₆alkyl, the latter two groups being optionally substituted by one or more substituents independently selected from halogen, C₃-C₇ cycloalkyl, OR⁶ and NR⁶R⁷, S(O)ₙR⁶ (where n = 0,1 or 2), CONR⁶R⁷, NR⁶COR⁷,SO₂NR⁶R⁷ and NR⁶SO₂R⁷;
or
R⁴ and R⁵ together with the nitrogen atom to which they are attached can form a 3-8 membered saturated heterocylic ring optionally containing one or more atoms selected from O, S(O)ₙ (where n = 0,1 or 2), NR⁸, and itself optionally substituted by halogen or C₁-₃ alkyl;
R⁶ and R⁷ independently represents a hydrogen atom or C₁-C₆ alkyl;
R⁸ is hydrogen, C₁-₄ alkyl, -COC₁-C₄ alkyl, CO₂C₁-C₄alkyl or CONR⁶C₁-C₄alkyl;
R⁹ represents aryl, heteroaryl, C₃-C₇ cycloalkyl or C₁₋₆alkyl, the latter two groups may be optionally substituted by one or more substituents independently selected from halogen, C₃-C₇ cycloalkyl, aryl, heteroaryl OR⁶ and NR⁶R⁷, S(O)ₙR⁶ (where n = 0, 1 or 2), CONR⁶R⁷, NR⁶COR⁷, SO₂NR⁶R⁷ and NR⁶SO₂R⁷;
R¹⁰ and R¹¹ independently represent aryl or heteroaryl, hydrogen, C₃-C₇ cycloalkyl or C₁₋₆alkyl, the latter two groups being optionally substituted by one or more substituents independently selected from halogen, C₃-C₇ cycloalkyl, aryl, heteroaryl, OR⁶ and NR⁶R⁷, S(O)ₙR⁶ (where n = 0, 1 or 2), CONR⁶R⁷, NR⁶COR⁷, SO₂NR⁶R⁷ and NR⁶SO₂R⁷;
or
R¹⁰ and R¹¹ together with the nitrogen atom to which they are attached can form a 3-8 membered saturated heterocylic ring optionally containing one or more atoms selected from O, S(O)ₙ (where n = 0, 1 or 2), NR⁸, and itself optionally substituted by halogen or C₁-C₃ alkyl.

2. A compound according to claim 1 in which R¹ and R² independently represent a hydrogen atom, C₂-C₆ alkenyl, C₂-C₆ alkynyl, C₃-C₇ cycloalkyl or a C₁₋₆alkyl group, the latter four groups being optionally substituted by one or more substituents independently selected from halogen, C₃-C₇ cycloalkyl, NR⁶R⁷, OR⁶, S(O)ₙR⁶ (where n is 0, 1 or 2) or R¹ and R² together can form a 3-8 membered ring optionally containing one or more atoms selected from O, S, NR⁶ and itself optionally substituted by one or more C₁-C₃ alkyl or halogen;

3. A compound according to claim 1 or 2 in which X is C₁₋₄alkyl or C₁₋₄alkoxy.

4. A compound according to any one of claims 1 to 3 in which Y is hydrogen.

5. A compound according to any one of claims 1 to 4 in which Z is phenyl or optionally substituted as defined in claim 1.

6. A compound according to any one of claims 1 to 4 in which Z is phenyl or optionally substituted by one or more substituents independently selected from halogen, C₁₋₃alkyl, cyano and SO₂R⁹.

7. A compound according to any one of claims 1 to 6 in which R¹ and R² are both hydrogen or one is hydrogen and the other is C₁₋₃ alkyl.

8. A compound according to any one of claims 1 to 7 selected from:
[(5-Methylbiphenyl-2-yl)oxy]acetic acid,
{[5-Ethyl-4'-(methylsulfonyl)biphenyl-2-yl]oxy} acetic acid
{[4'-(Ethylsulfonyl)-5-methoxybiphenyl-2-yl]oxy}acetic acid
[[4-Chloro-4'-(ethylsulfonyl)-2',5-dimethyl[1,1'-biphenyl]-2-yl]oxy]-acetic acid
[[4'-(Ethylsulfonyl)-2',5-dimethyl[1,1'-biphenyl]-2-yl]oxy]-acetic acid
2-[[3'-Cyano-5-methyl[1,1'-biphenyl]-2-yl]oxy]-(2S)-propanoic acid
2-[[2'-Fluoro-5'-cyano-5-methyl[1,1'-biphenyl]-2-yl]oxy]-(2S)-propanoic acid
and pharmaceutically acceptable salts thereof.

9. A compound of formula (I) according to any one of claims 1 to 8 for use in therapy.

10. Use of a compound of formula (I), or a pharmaceutically acceptable salt thereof, as defined in claims 1 to 8 in the manufacture of a medicament for use in the treatment of a disease mediated by prostaglandin D2.

11. Use of a compound of formula (I), or a pharmaceutically acceptable salt thereof, as defined in claims 1 to 8 in the manufacture of a medicament for use in the treatment of a respiratory disease, such as asthma and rhinitis, in a patient suffering from, or at risk of, said disease.

## Patentansprüche

1. Verbindungen der Formel (I) und deren pharmazeutisch annehmbaren Salze: 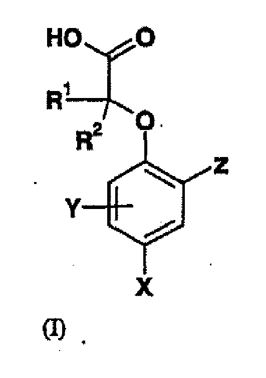 wobei:
X für C₁₋₆-Alkyl oder OR⁶ steht;
Y ausgewählt ist aus Wasserstoff, Halogen, CN, Nitro, SO₂R³, OR⁴, SR⁴, SOR³, SO₂NR⁴R⁵, CONR⁴R⁵, NR⁴R⁵, NR⁶SO₂R³, NR⁶CO₂R⁶, NR⁶COR³, C₂-C₆-Alkenyl, C₂-C₆-Alkinyl, C₃-C₇-Cycloalkyl oder C₁₋₆-Alkyl, wobei die vier letztgenannten Gruppen gegebenenfalls substituiert sind durch einen oder mehrere Substituenten unabhängig voneinander ausgewählt aus Halogen, OR⁶ und NR⁶R⁷, S(O)ₙR⁶, wobei n für 0, 1 oder 2 steht;
Z für Aryl oder einen Ring A steht, wobei A für einen sechsgliedrigen heterocyclischen aromatischen Ring mit einem oder mehreren Stickstoffatomen steht oder für einen 6,6- oder 6,5-kondensierten Bicyclus mit einem oder mehreren Sauerstoff-, Stickstoff-, Schwefelatomen stehen kann, wobei die Arylgruppe oder A-Ringe jeweils gegebenenfalls substituiert sind durch einen oder mehrere Substituenten unabhängig voneinander ausgewählt aus Wasserstoff, Halogen, CN, OH, SH, Nitro, COR⁹, CO₂R⁶, SO₂R⁹, OR⁹, SR⁹, SOR⁹, SO₂NR¹⁰R¹¹, CONR¹⁰R¹¹, NR¹⁰R¹¹, NHSO₂R⁹, NR⁹SO₂R⁹, NR⁶CO₂R⁶, NHCOR⁹, NR⁹COR⁹, NR⁶CONR⁴R⁵, NR⁶SO₂NR⁴R⁵, Aryl, Heteroaryl, C₂-C₆-Alkenyl, C₂-C₆-Alkinyl, C₃-C₇-Cycloalkyl oder C₁₋C₆-Alkyl, wobei die vier letztgenannten Gruppen gegebenenfalls substituiert sind durch einen oder mehrere Substituenten unabhängig voneinander ausgewählt aus Halogen, C₃-C₇-Cycloalkyl, OR⁶, NR⁶R⁷, S(O)ₙR⁶ (wobei n für 0, 1 oder 2 steht), CONR⁶R⁷, NR⁶COR⁷, SO₂NR⁶R⁷ und NR⁶SO₂R⁷;
R¹ und R² unabhängig voneinander für ein Wasserstoffatom, Halogen, C₂-C₆-Alkenyl, C₂-C₆-Alkinyl, C₃-C₇-Cycloalkyl oder eine C₁₋₆-Alkylgruppe stehen, wobei die vier letztgenannten Gruppen gegebenenfalls substituiert sind durch einen oder mehrere Substituenten unabhängig voneinander ausgewählt aus Halogen, C₃-C₇-Cycloalkyl, NR⁶R⁷, OR⁶, S(O)ₙR⁶ (wobei n für 0, 1 oder 2 steht);
oder
R¹ und R² zusammen einen 3- bis 8-gliedrigen Ring bilden können, der gegebenenfalls ein oder mehrere Atome ausgewählt aus O, S, NR⁶ enthält und selbst gegebenenfalls durch einen oder mehrere C₁-C₃-Alkyl-oder Halogengruppen substituiert ist;
R³ für C₃-C₇-Cycloalkyl oder C₁₋₆-Alkyl, das gegebenenfalls substituiert ist durch einen oder mehrere Substituenten unabhängig voneinander ausgewählt aus Halogen, C₃-C₇-Cycloalkyl, OR⁶ und NR⁶R⁷, S(O)ₙR⁶ (wobei n = 0, 1 oder 2), CONR⁶R⁷, NR⁶COR⁷, SO₂NR⁶R⁷ und NR⁶SO₂R⁷, steht;
R⁴ und R⁵ unabhängig voneinander für Wasserstoff, C₃-C₇-Cycloalkyl oder C₁₋₆-Alkyl stehen, wobei die beiden letztgenannten Gruppen gegebenenfalls substituiert sind durch einen oder mehrere Substituenten unabhängig voneinander ausgewählt aus Halogen, C₃-C₇-Cycloalkyl, OR⁶ und NR⁶R⁷, S(O)ₙR⁶ (wobei n = 0, 1 oder 2), CONR⁶R⁷, NR⁶COR⁷, SO₂NR⁶R⁷ und NR⁶SO₂R⁷;
oder
R⁴ und R⁵ zusammen mit dem Stickstoffatom, an das sie gebunden sind, einen 3- bis 8-gliedrigen gesättigten heterocyclischen Ring bilden können, der gegebenenfalls ein oder mehrere Atome ausgewählt aus O, S(O)ₙ (wobei n = 0, 1 oder 2), NR⁸ enthält und selbst gegebenenfalls durch Halogen oder C₁₋₃-Alkyl substituiert ist;
R⁶ und R⁷ unabhängig voneinander für ein Wasserstoffatom oder C₁₋C₆-Alkyl stehen;
R⁸ für Wasserstoff, C₁₋₄-Alkyl, -COC₁-C₄-Alkyl, CO₂-C₁-C₄-Alkyl oder CONR⁶-C₁-C₄-Alkyl steht;
R⁹ für Aryl, Heteroaryl, C₃-C₇-Cycloalkyl oder C₁₋₆-Alkyl steht, wobei die beiden letztgenannten Gruppen gegebenenfalls substituiert sind durch einen oder mehrere Substituenten unabhängig voneinander ausgewählt aus Halogen, C₃-C₇-Cycloalkyl, Aryl, Heteroaryl, OR⁶ und NR⁶R⁷, S(O)ₙR⁶ (wobei n = 0, 1 oder 2), CONR⁶R⁷, NR⁶COR⁷, SO₂NR⁶R⁷ und NR⁶SO₂R⁷ steht;
R¹⁰ und R¹¹ unabhängig voneinander für Aryl oder Heteroaryl, Wasserstoff, C₃-C₇-Cycloalkyl oder C₁₋₆-Alkyl stehen, wobei die beiden letztgenannten Gruppen gegebenenfalls substituiert sind durch einen oder mehrere Substituenten unabhängig voneinander ausgewählt aus Halogen, C₃-C₇-Cycloalkyl, Aryl, Heteroaryl, OR⁶ und NR⁶R⁷, S(O)ₙR⁶ (wobei n = 0, 1 oder 2), CONR⁶R⁷, NR⁶COR⁷, SO₂NR⁶R⁷ und NR⁶SO₂R⁷ steht;
oder
R¹⁰ und R¹¹ zusammen mit dem Stickstoffatom, an das sie gebunden sind, einen 3- bis 8-gliedrigen gesättigten heterocyclischen Ring bilden können, der gegebenenfalls ein oder mehrere Atome ausgewählt aus O, S(O)ₙ (wobei n = 0, 1 oder 2), NR⁸ enthält und selbst gegebenenfalls durch Halogen oder C₁-C₃-Alkyl substituiert ist.

2. Verbindungen nach Anspruch 1, wobei R¹ und R² unabhängig voneinander für ein Wasserstoffatom, C₂-C₆-Alkenyl, C₂-C₆-Alkinyl, C₃-C₇-Cycloalkyl oder eine C₁-C₆-Alkylgruppe stehen, wobei die vier letztgenannten Gruppen gegebenenfalls substituiert sind durch einen oder mehrere Substituenten unabhängig voneinander ausgewählt aus Halogen, C₃-C₇-Cycloalkyl, NR⁶R⁷, OR⁶, S(O)ₙR⁶ (wobei n = 0, 1 oder 2) oder R¹ und R² zusammen einen 3- bis 8-gliedrigen Ring bilden können, der gegebenenfalls ein oder mehrere Atome ausgewählt aus O, S, NR⁶ enthält und selbst gegebenenfalls durch einen oder mehrere C₁-C₃-Alkyl- oder Halogengruppen substituiert ist.

3. Verbindungen nach Anspruch 1 oder 2, wobei X für C₁₋₄-Alkyl oder C₁₋₄-Alkoxy steht.

4. Verbindungen nach einem der Ansprüche 1 bis 3, wobei Y für Wasserstoff steht.

5. Verbindungen nach einem der Ansprüche 1 bis 4, wobei Z für Phenyl steht oder gegebenenfalls wie in Anspruch 1 definiert substituiert ist.

6. Verbindungen nach einem der Ansprüche 1 bis 4, wobei Z für Phenyl steht oder gegebenenfalls substituiert ist durch einen oder mehrere Substituenten unabhängig voneinander ausgewählt aus Halogen, C₁₋₃-Alkyl, Cyano und SO₂R⁹.

7. Verbindungen nach einem der Ansprüche 1 bis 6, wobei R¹ und R² beide für Wasserstoff stehen oder der eine Rest für Wasserstoff steht und der andere für C₁₋₃-Alkyl steht.

8. Verbindungen nach einem der Ansprüche 1 bis 7, ausgewählt aus:
[(5-Methylbiphenyl-2-yl)oxy]essigsäure
{[5-Ethyl-4'-(methylsulfonyl)biphenyl-2-yl]oxy}essigsäure
{[4'-(Ethylsulfonyl)-5-methoxybiphenyl-2-yl]oxy}essigsäure
[[4-Chlor-4'-(ethylsulfonyl)-2',5-dimethyl[1,1'-biphenyl]-2-yl]oxy]essigsäure
[[4'-(Ethylsulfonyl)-2',5-dimethyl[1,1'-biphenyl]-2-yl]oxy]essigsäure
2-[[3'-Cyano-5-methyl[1,1'-biphenyl]-2-yl]oxy]-(2S)-propansäure
2-[[2'-Fluor-5'-cyano-5-methyl[1,1'-biphenyl]-2-yl]oxy]-(2S)-propansäure
und deren pharmazeutisch annehmbaren Salze.

9. Verbindungen der Formel (I) nach einem der Ansprüche 1 bis 8 zur Verwendung in der Therapie.

10. Verwendung einer Verbindung der Formel (I) oder eines pharmazeutisch annehmbaren Salzes davon nach einem der Ansprüche 1 bis 8 bei der Herstellung eines Medikaments zur Verwendung bei der Behandlung einer durch Prostaglandin D2 vermittelten Krankheit.

11. Verwendung einer Verbindung der Formel (I) oder eines pharmazeutisch annehmbaren Salzes davon nach einem der Ansprüche 1 bis 8 bei der Herstellung eines Medikaments zur Verwendung bei der Behandlung einer Atemwegserkrankung wie Asthma und Rhinitis bei einem Patienten, der an dieser Krankheit leidet bzw. bei dem das Risiko dieser Krankheit besteht.

## Revendications

1. Composé de formule (I) ou sel pharmaceutiquement acceptable de celui-ci : 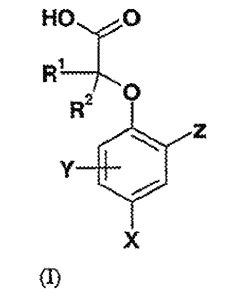 dans laquelle
X représente alkyle en C₁₋₆ ou OR⁶ ;
Y est choisi parmi hydrogène, halogène, CN, nitro, SO₂R³, OR⁴, SR⁴, SOR³, SO₂NR⁴R⁵, CONR⁴R⁵, NR⁴R⁵, NR⁶SO₂R³, NR⁶CO₂R⁶, NR⁶COR³, alcényle en C₂-C₆, alcynyle en C₂-C₆, cycloalkyle en C₃-C₇ ou alkyle en C₁₋₆, les quatre derniers groupes étant éventuellement substitués par un ou plusieurs substituants choisis indépendamment parmi halogène, OR⁶ et NR⁶R⁷, S(O)ₙR⁶ où n vaut 0, 1 ou 2 ;
Z représente aryle ou un cycle A, où A est un cycle aromatique hétérocyclique à six chaînons contenant un ou plusieurs atomes d'azote ou peut être un bicycle condensé en 6,6 ou en 6,5 contenant un ou plusieurs atomes O, N, S, les cycles aryle ou A étant tous éventuellement substitués par un ou plusieurs substituants choisis indépendamment parmi hydrogène, halogène, CN, OH, SH, nitro, COR⁹, CO₂R⁶, SO₂R⁹, OR⁹, SR⁹, SOR⁹, SO₂NR¹⁰R¹¹, CONR¹⁰R¹¹, NR¹⁰R¹¹, NHSO₂R⁹, NR⁹SO₂R⁹, NR⁶CO₂R⁶, NHCOR⁹, NR⁹COR⁹, NR⁶CONR⁴R⁵, NR⁶SO₂NR⁴R⁵, aryle, hétéroaryle, alcényle en C₂-C₆, alcynyle en C₂-C₆, cycloalkyle en C₃-C₇ ou alkyle en C₁₋₆, les quatre derniers groupes étant éventuellement substitués par un ou plusieurs substituants choisis indépendamment parmi halogène, cycloalkyle en C₃-C₇, OR⁶, NR⁶R⁷, S(O)ₙR⁶ (où n vaut 0, 1 ou 2), CONR⁶R⁷, NR⁶COR⁷, SO₂NR⁶R⁷ et NR⁶SO₂R⁷ ;
R¹ et R² représentent, indépendamment, un atome d'hydrogène, halogène, alcényle en C₂-C₆, alcynyle en C₂-C₆, cycloalkyle en C₃-C₇ ou alkyle en C₁₋₆, les quatre derniers groupes étant éventuellement substitués par un ou plusieurs substituants choisis indépendamment parmi halogène, cycloalkyle en C₃-C₇, NR⁶R⁷, OR⁶, S(O)ₙR⁶ (où n vaut 0, 1 ou 2) ;
ou
R¹ et R² ensemble peuvent former un cycle de 3 à 8 membres contenant éventuellement un ou plusieurs atomes choisis parmi O, S, NR⁶ et lui-même étant éventuellement substitué par un ou plusieurs groupes alkyle en C₁-C₃ ou par halogène ;
R³ représente un groupe cycloalkyle en C₃-C₇ ou alkyle en C₁₋₆ qui peut éventuellement être substitué par un ou plusieurs substituants choisis indépendamment parmi halogène, cycloalkyle en C₃-C₇, OR⁶ et NR⁶R⁷, S(O)ₙR⁶ (où n = 0, 1 ou 2), CONR⁶R⁷, NR⁶COR⁷, SO₂NR⁶R⁷ et NR⁶SO₂R⁷ ;
R⁴ et R⁵ représentent, indépendamment, hydrogène, cycloalkyle en C₃-C₇ ou alkyle en C₁₋₆, les deux derniers groupes étant éventuellement substitués par un ou plusieurs substituants choisis indépendamment parmi halogène, cycloalkyle en C₃-C₇, OR⁶ et NR⁶R⁷, S(O)ₙR⁶ (où n = 0, 1 ou 2), CONR⁶R⁷, NR⁶COR⁷, SO₂NR⁶R⁷ et NR⁶SO₂R⁷ ;
ou
R⁴ et R⁵ ensemble avec l'atome d'azote auquel ils sont attachés peuvent former un cycle saturé hétérocyclique de 3 à 8 membres contenant éventuellement un ou plusieurs atomes choisis parmi les O, S(O)ₙ, (où n = 0, 1 ou 2), NR⁸ et lui-même étant éventuellement substitué par halogène ou alkyle en C₁₋₃ ;
R⁶ et R⁷ représentent, indépendamment, un atome d'hydrogène ou alkyle en C₁₋₆;
R⁸ représente hydrogène, un groupe alkyle en C₁₋₄, -CO(alkyle en C₁-C₄) ou CO₂(alkyle en C₁-C₄) ou CONR⁶(alkyle en C₁-C₄) ;
R⁹ représente aryle, hétéroaryle, cycloalkyle en C₃-C₇ ou alkyle en C₁₋₆, les deux derniers groupes pouvant éventuellement être substitués par un ou plusieurs substituants choisis indépendamment parmi halogène, cycloalkyle en C₃-C₇, aryle, hétéroaryle, OR⁶ et NR⁶R⁷, S(O)ₙR⁶ (où n = 0, 1 ou 2), CONR⁶R⁷, NR⁶COR⁷, SO₂NR⁶R⁷ et NR⁶SO₂R⁷ ;
R¹⁰ et R¹¹ représentent, indépendamment, aryle ou hétéroaryle, hydrogène,cycloalkyle en C₃-C₇ ou alkyle en C₁₋₆, ces deux derniers groupes étant éventuellement substitués par un ou plusieurs substituants choisis indépendamment parmi halogène, cycloalkyle en C₃-C₇, aryle, hétéroaryle, OR⁶ et NR⁶R⁷, S(O)ₙR⁶ (où n = 0, 1 ou 2), CONR⁶R⁷, NR⁶COR⁷, SO₂NR⁶R⁷ et NR⁶SO₂R⁷ ;
ou
R¹⁰ et R¹¹ ensemble avec l'atome d'azote auquel ils sont attachés peuvent former un cycle saturé hétérocyclique de 3 à 8 membres contenant éventuellement un ou plusieurs atomes choisis parmi O, S(O)ₙ, (où n = 0, 1 ou 2), NR⁸ et lui-même étant éventuellement substitué par halogène ou alkyle en C₁-C₃.

2. Composé selon la revendication 1, où R¹ et R² représentent, indépendamment, un atome d'hydrogène, alcényle en C₂-C₆, alcynyle en C₂-C₆, cycloalkyle en C₃-C₇ ou alkyle en C₁₋₆, les quatre derniers groupes étant éventuellement substitués par un ou plusieurs substituants choisis indépendamment parmi halogène, cycloalkyle en C₃-C₇, NR⁶R⁷, OR⁶, S(O)ₙR⁶ (ou n représente 0, 1 ou 2) ou R¹ et R² ensemble peuvent former un cycle de 3-8 chaînons contenant éventuellement un ou plusieurs atomes choisis parmi O, S, NR⁶ et lui-même étant éventuellement substitué par un ou plusieurs groupes alkyle en C₁-C₃ ou par halogène.

3. Composé selon la revendication 1 où 2, où X représente un groupe alkyle en C₁₋₄ ou alcoxy en C₁₋₄.

4. Composé selon l'une quelconque des revendications 1 à 3, où Y représente hydrogène.

5. Composé selon l'une quelconque des revendications 1 à 4, où Z représente phényle ou est éventuellement substitué comme défini dans la revendication 1.

6. Composé selon l'une quelconque des revendications 1 à 4, où Z représente phényle ou est éventuellement substitué par un ou plusieurs substituants choisis indépendamment parmi halogène, alkyle en C₁₋₃, cyano et SO₂R⁹.

7. Composé selon l'une quelconque des revendications 1 à 6, où R¹ et R² représentent tous les deux hydrogène ou un représente hydrogène et l'autre représente alkyle en C₁₋₃.

8. Composé selon l'une quelconque des revendications 1 à 7, choisi parmi :
l'acide [(5-méthylbiphényl-2-yl)oxy]acétique,
l'acide {[5-éthyl-4'-(méthylsulfonyl)biphényl-2-yl]oxy}acétique
l'acide {[4'-(éthylsulfonyl)-5-méthoxybiphényl-2-yl]oxy}acétique
l'acide [[4-chloro-4'-(éthylsulfonyl)-2',5-diméthyl[1,1'-biphényl]-2-yl]oxy]acétique
l'acide [[4'-(éthylsulfonyl)-2',5-diméthyl[1,1'-biphényl]-2-yl]oxy]acétique
l'acide 2-[[3'-cyano-5-méthyl[1,1'-biphényl]-2-yl]oxy]-(2S)-propanoïque
l'acide 2-[[2'-fluoro-5'-cyano-5-méthyl[1,1'-biphényl]-2-yl]oxy]-(2S)-propanoïque et les sels pharmaceutiquement acceptables de ceux-ci.

9. Composition de formule (I) selon l'une quelconque des revendications 1 à 8 destinée à une utilisation en thérapie.

10. Utilisation d'un composé de formule (I), ou d'un sel pharmaceutiquement acceptable de celui-ci, tel que défini dans les revendications 1 à 8 dans la préparation d'un médicament destiné à une utilisation dans le traitement d'une maladie dans laquelle intervient la prostaglandine D2.

11. Utilisation d'un composé de formule (I), ou d'un sel pharmaceutiquement acceptable de celui-ci, tel que défini dans les revendications 1 à 8 dans la préparation d'un médicament destiné à une utilisation dans le traitement d'une maladie respiratoire, telle que l'asthme et la rhinite et chez un patient souffrant de ou risquant de souffrir de ladite maladie.
